# EUROPEAN PATENT SPECIFICATION

(11) **EP 4 188 282 B1**
(45) Date of publication and mention of the grant of the patent: **09.04.2025**
(21) Application number: 22817051.0
(22) Date of filing: 19.05.2022
(51) Int. Cl.: A61F 2/24

(54) **PROSTHETIC TRANSCATHETER HEART VALVE (THV) SYSTEM**
PROTHETISCHES TRANSKATHETERHERZKLAPPENSYSTEM
SYSTÈME DE VALVE CARDIAQUE TRANSCATHÉTER (THV) PROTHÉTIQUE

(30) Priority: 18.10.2021 IN 202121047196
(43) Date of publication of application: 07.06.2023
(73) Proprietor: Meril Life Sciences Pvt Ltd, Gujarat, Vapi 396191 (IN)
(72) Inventor: BHATT, Sanjeev Nauttam, Mumbai 400064 (IN); PARMAR, Harshad Amrutlal, Vapi 396191 (IN)
(74) Representative: Marks & Clerk LLP
(86) International application number: PCT/IN2022/050475
(87) International publication number: WO 2023/067614

(56) References cited:
- WO-A1-2020/061124
- CN-A- 103 228 232
- US-A1- 2018 289 476
- US-B2- 9 962 258

## Description

### FIELD OF INVENTION

The present invention relates to a prosthetic system. More specifically, the present invention relates to a prosthetic trans-catheter heart valve system.

### BACKGROUND

The function of a prosthetic heart valve is to replace a diseased native heart valve. The replacement procedure may be surgical (using open heart surgery) or percutaneous.

In the surgical procedure, leaflets of the native valve are excised and the annulus is sculpted to receive a prosthetic valve. For many years, the definitive treatment for such disorders was the surgical repair or replacement of the valve during open heart surgery, but such surgeries are prone to many complications. Some patients do not survive the surgical procedure due to the trauma associated with the procedure and duration of the extracorporeal blood circulation. Due to this, a number of patients are deemed inoperable and hence remain untreated.

Against the surgical procedure, a percutaneous catheterization technique has been developed for introducing and implanting a prosthetic heart valve using a flexible catheter that is considerably less invasive than an open-heart surgery. In this technique, a prosthetic valve is mounted by crimping on a balloon located at the distal end of a flexible catheter. The catheter is most commonly introduced into a blood vessel usually through a peripheral artery (rarely via a vein); most likely a common femoral or sometimes axillary or carotid artery of the patient or rarely via a transapical route amongst other access routes. The catheter with the prosthetic valve crimped on the balloon is then advanced through the blood vessel till the crimped valve reaches the implantation site. The valve is allowed to expand to its functional size at the site of the defective native valve by inflating the balloon on which the valve is mounted. Alternatively, the valve may have a self-expanding stent or support frame that expands the valve to its functional size by withdrawing the restricting sheath mounted over the valve. The former prosthetic valve is termed as a "balloon-expandable" valve and the latter as a "self-expanding" valve.

Both the balloon-expandable and self-expandable valves incorporate a support frame or a stent that is typically a tubular scaffold structure and a plurality of leaflets typically three leaflets.

The design of the support frame plays an important role in the performance of the prosthetic valve. For achieving long term performance of the prosthetic valve, the support frame should have adequate radial strength to resist radially collapsing or compressive arterial forces. The support frame should also have adequate fatigue resistance to resist arterial cyclic forces imposed by opening and closing of the prosthetic valve during systolic and diastolic cycles. In view of these requirements, the design of the support frame of a transcatheter prosthetic heart valve should be based on structural robustness, sufficient radial strength or stiffness, and high fatigue strength. Further, the size and/or axial length of the support frame is desirably optimized to ensure enhanced interface with the native anatomy.

Reference is made to the patent application for trans-catheter aortic prosthetic heart valve (THV) which is published as WO 2018/109779A1 and US 2018/0289476. The THV made using the configuration disclosed in the aforementioned application has large size matrix - conventional, intermediate and extra-large sizes and it is directly crimped on the balloon of the delivery system.

The THV system disclosed in the above applications addresses several unmet clinical needs viz.:
1. Severe aortic valve stenosis (intermediate to high surgical risk i.e. Society of Thoracic Surgeons (STS) risk level ≥ 4%).
2. Low surgical risk population with STS risk level <4%.
3. Symptomatic moderate aortic valve stenosis
4. Patients presenting with low flow, low gradient
5. Implantation in younger patient population (<65 years).
6. Compatibility with bicuspid aortic valve anatomy.
7. Compatibility with highly calcified aortic root complex.
8. Compatibility with valve-in-valve interventions viz. TAVR-in-SAVR and TAVR-in-TAVR,
9. Compatibility with pulmonary valve replacement.
10. Treatment of aortic regurgitation (AR).
11. Treatment of asymptotic severe aortic stenosis.

The frame of the preferred embodiment of the THV disclosed in the aforementioned invention has three rows of hexagonal cells.

Further, in case of balloon expandable prosthetic heart valves, they are delivered to the implantation site by a balloon catheter. The delivery system, i.e. balloon catheter also plays a very important role in accurately identifying the deployment zone where the prosthetic heart valve is implanted by expanding the balloon. There is a continued need to ensure optimal and accurate placement and precise deployment of the valve at desired implantation site in a patient.
US 2018/289476 describes a prosthetic valve having fluoroscopic properties.

Therefore, in light of the above, a need exists for a stented transcatheter prosthetic heart valve system which overcomes the drawbacks of the existing systems.

### SUMMARY

The present invention relates to a prosthetic aortic valve which is radially expandable and collapsible and suitable for mounting on a balloon of a delivery catheter in a radially collapsed condition as defined by claim 1. The present invention as described in the following description ingeniously retains the core legacy technology of the THV disclosed in the aforementioned patent application provided in the background with several enhancements incorporated.

Accurate placement and precise deployment of a prosthetic valve in aorta is very important to achieve optimal performance i.e. reduced valve gradients (sustained hemodynamics), absence of paravalvular regurgitation and avoiding any iatrogenic damage to conduction system that necessitates a new permanent pacemaker implantation. The ideal location of implantation of a prosthetic valve in aorta is preferably the orthotopic position where the attempt is to superimpose the prosthetic annulus (neo-annulus) to the native annulus ring. Implanting the prosthetic valve at this location has three important advantages as outlined below.

One advantage is better anatomical placement of the prosthetic valve. The annulus of the native valve and its leaflets are stenosed and may also be calcified. When a prosthetic valve, sized to match the native annulus, is expanded at the orthotopic position, the frame is held firmly within the stenosed annulus which may have calcified leaflets, thereby offering geographical fix which eliminates risk of embolization of the prosthetic valve by dislodgement.

The second advantage is minimal protrusion of valve in left ventricle. It is important to deploy prosthetic valve at its annular position and not too deep towards ventricular end, also known as infra-annular position due to two important sub-aortic anatomical zones. First zone is the membranous septum which has densely populated cardiac conduction musculature (AV node) which transfer electrical impulses to maintain normal heart rhythm. It is important that the prosthetic valve does not dwell into the left ventricle outflow tract (LVOT) thereby not disturbing cardiac conduction system. The second zone is the aortomitral curtain and position of native mitral valve which is located posterolaterally to the aortic valve. Wrongly positioned prosthetic valve may have the propensity to interfere with normal functioning of the anterior leaflet of the mitral valve, thus impacting the functioning of the mitral valve. A prosthetic valve implanted by the method recommended herein (which method is not presently claimed) achieves minimum protrusion of the prosthetic valve in LVOT.

The third advantage is minimizing obstruction to ostia of coronary arteries which are located along the coronary sinus of valsalva or may be above the sino-tubular junction. Ideally, the prosthetic valve should not obstruct the blood flow into these arteries by obstructing or causing 'jailing' of their ostia. The precise location of the prosthetic valve at orthotopic position prevents this by minimizing the protrusion of the frame 101 into the ascending aorta. The jailing of the ostia of coronary arteries is avoided in the present invention by large uncovered cells at the outflow end and the short frame height of the expanded prosthetic valve of this invention.

This invention allows the prosthetic aortic valve to be accurately positioned and precisely deployed at the orthotopic position. This is achieved by design of the structure of the prosthetic aortic valve and the delivery system of this invention. The prosthetic aortic valve and the delivery system of are described below.

The prosthetic aortic valve is radially expandable and collapsible and is suitable for mounting on a balloon of a delivery catheter in a radially collapsed condition. The prosthetic valve includes a radially collapsible and expandable support frame having a distal end, a proximal end and three circumferentially extending rows of angled struts having an upper row at the distal end of the frame, a lower row at the proximal end of the frame and a middle row located between the proximal row, and the distal row where, a distal position refers to a position away from the operator. The lower row is towards the inflow end of the support frame. The rows of angled struts have an undulating shape with peaks and valleys, the peaks of the upper row of angled struts face the valleys of the middle row of angled struts, and the peaks of the middle row of angled struts face the valleys of the lower row of angled struts. The rows of angled struts are connected to each other to form the support frame including two adjacently placed rows of cells between its distal end and the proximal end. The valleys of the upper row of angled struts are connected to the corresponding peaks of the middle row of angled struts by links where a link is either a diamond shaped cell or a rhombus body (with/without holes), thereby forming an upper row of cells that includes interlaced octagonal cells creating alternate sequence of rhombus bodies (with/without holes) or diamond shaped cells at each junction. The diamond shaped cells have open structure, while the rhombus body has a solid structure. The valleys of the middle row of angled struts are connected to the corresponding peaks of the lower row of angled struts by links where a link is either a diamond shaped cell (with open structure) or a rhombus body (with solid structure), thereby forming a lower row of cells having interlaced octagonal cells creating alternate sequence of rhombus bodies or diamond shaped cells at each junction.

The reduction in number of rows and the specific shape of the cells results in reduced foreshortening of the frame on radial expansion which makes it easier for the operator to implant the prosthetic heart valve accurately.

The two rows of cells include an upper row disposed towards the outflow end of the support frame and a lower row of cells is disposed towards the inflow end of the support frame. The upper row of cells includes three solid rhombus bodies with holes, spaced angularly at 120° with respect to each other, forming three commissure attachment areas where the commissure areas or tabs of the two adjacent leaflets are attached.

In an embodiment, the prosthetic aortic valve includes three circumferentially extending rows of angled struts forming two rows of cells, and a plurality of links. Each link includes either a diamond shaped cell or a rhombus body (with/without holes).

Any two consecutive angled struts of a circumferentially extending row of angled struts form a peak or a valley. The peaks of the angled struts of one circumferentially extending row of the angled struts face the valleys of the angled struts of an adjacent circumferentially extending row of the angled struts. The valleys of the angled struts of the one circumferentially extending row are connected to the corresponding peaks of the angled struts of the adjacent circumferentially extending row of angled struts via the links. Thus, a peak in one circumferentially extending row of angled struts has a corresponding valley in the adjacent circumferentially extending row of angled struts facing each other. Similarly, a valley in one circumferentially extending row of angled struts has a corresponding peak in the adjacent circumferentially extending row of angled struts facing each other.

The interconnection of the one row and the adjacent row of angled struts via the links results in a cell structure having interlaced octagonal cells and diamond shaped cells or solid rhombus bodies. Three of the links of the upper row of cells, spaced angularly at 120° with respect to each other, are rhombus bodies provided with holes, forming three commissure attachment areas.

Three leaflets made from a biocompatible material with sufficient flexibility are provided to allow unidirectional flow of blood from inflow end of the prosthetic aortic valve to the outflow end and prevent the flow of blood in reverse direction by opening and closing the leaflets during systolic and diastolic cycles.

The prosthetic aortic valve includes an internal skirt made of a biocompatible material covering internal surface of the lower row of cells at least partially. An external skirt made of a biocompatible material is also provided, covering external surface of the lower row of cells at least partially. The external skirt has excess material such that the external skirt forms a slack when the support frame is in a radially expanded condition and the slack reduces when the support frame is in the radially collapsed condition.

The delivery system includes a balloon catheter comprising an elongated shaft having a distal end and a proximal end. An inflatable balloon is attached to the distal end of the elongated shaft, and a handle attached to the proximal end of the elongated shaft. In addition, the delivery system includes other components required for a balloon catheter. The distal end refers to the end away from the operator.

Four radiopaque markers (a distal marker, a proximal marker, a middle marker and a landing zone marker) are provided on a portion of the shaft of the balloon catheter that is located within the balloon. The distal marker is located towards the distal end of the balloon, the proximal marker is located towards the proximal end of the balloon. The middle marker is located between the proximal and distal markers equidistant from the distal and proximal markers, and the landing zone marker is located between the distal marker and the middle marker at specific distance from the distal marker.

The above prosthetic aortic valve and the balloon catheter form an assembly. The prosthetic aortic valve has fluoroscopic properties and when crimped on a balloon of the balloon catheter between two stoppers and two extreme radiopaque markers (i.e. proximal marker and distal marker), exhibits alternate light and dense areas when viewed under fluoroscopy. The dense areas are formed by circumferentially extending rows of angled struts and light areas are formed by the crooked struts of the diamond shaped cells or by the rhombus bodies and the commissure areas.

The landing zone marker of the delivery catheter is located behind the mid-point of the light area towards the inflow end of the prosthetic aortic valve.

As mentioned above, the devices of the present invention allow accurate positioning and precise deployment of the prosthetic aortic valve at the orthotopic position. The first step of deployment (not claimed) includes introducing an introducer sheath into the vasculature of a patient and subsequently, introducing and navigating a standard angiographic Pig-Tail Catheter through the introducer sheath into the patient's vasculature and parking its distal end at the lowest end within the non-coronary cusp under fluoroscopic guidance.

A standard recommended guidewire is then introduced under fluoroscopic guidance and navigating it beyond the aortic orifice of the patient. Post that, the prosthetic aortic valve, pre-crimped on the balloon of the delivery catheter, is introduced through the introducer sheath and is navigated to the aortic orifice of the patient by guiding it over the guidewire under fluoroscopic guidance.

An accurate positioning of the prosthetic aortic valve at the annular plane is then attained by coinciding the centre of the landing zone marker within the balloon of the delivery catheter with the lower end of the pigtail, and the center of the light area towards the inflow zone with the lower end of the pigtail. The prosthetic aortic valve is deployed at this position by inflating the balloon of the delivery catheter. The balloon of the delivery catheter is then deflated after implanting the prosthetic aortic valve and the delivery catheter shaft along with the balloon is withdrawn from the patient's vasculature.

The foregoing features and other features as well as the advantages of the invention will become more apparent from the following detailed description, which proceeds with reference to the accompanying figures. References to methods of treatment of the human or animal body described herein are not claimed. In particular, the references to the method of accurately positioning and deploying a prosthetic aortic valve at an annular plane as described in para. [0030] - [0032] and para [00131] - [00138] of this description are to be interpreted as references to the prosthetic aortic valves, delivery catheters and assemblies of the present invention for use in that method.

### BRIEF DESCRIPTION OF DRAWINGS

The summary above, as well as the following detailed description of illustrative embodiments, is better understood when read in conjunction with the appended figures. For the purpose of illustrating the present disclosure, various exemplary embodiments are shown in the figures. However, the disclosure is not limited to the description and figures disclosed herein. Moreover, those familiar with the art will understand that the figures are not to scale. Wherever possible, like elements have been indicated by identical numbers.
FIGs. 1 and 1b show exploded views of the frame 101 in accordance with an embodiment of the present disclosure.
FIGs. 1a shows the commissure areas 101d of the frame 101 in accordance with an embodiment of the present disclosure.
FIG. 1c illustrates an exploded view of the frame 101 in accordance with another embodiment of the present disclosure.
FIGs. 1d-1g illustrate exploded views of the frame 101 in accordance with different embodiments of the present disclosure.
FIG. 2 illustrates a leaflet 103 in accordance with an embodiment of the present disclosure.
Fig. 2a illustrates a leaflet 103x in accordance with another embodiment of the present disclosure.
FIG. 3 illustrates a prosthetic valve 100 with an internal skirt 105 in accordance with an embodiment of the present disclosure.
FIG. 4 illustrates THV 100 with an external skirt 107 in accordance with an embodiment of the present disclosure.
FIG. 5 depicts a delivery catheter 200 in accordance with an embodiment of the present disclosure.
FIG. 6 illustrates an exploded view of the balloon 201 with support tube 207 in accordance with an embodiment of the present disclosure.
FIG. 7 shows the implanted THV 100 at the aortic annulus in accordance with an embodiment of the present disclosure.
FIG. 8 depicts schematically the THV 100 made using the frame 101 of FIGs. 1 and 1b mounted over the balloon of the delivery system 200 in a crimped condition as visible under fluoroscopy in accordance with an embodiment of the present disclosure.
FIG. 9 depicts schematically the THV 100 made using the frame 101 of FIG. 1c mounted over the balloon of the delivery system 200 in a crimped condition as visible under fluoroscopy in accordance with an embodiment of the present disclosure.
FIGs. 10-11 show a native aortic root complex schematically.
FIG. 12 discloses a method of implantation of the THV 100, which method is not claimed.
FIGs. 13-14 illustrate the positioning of the THV 100 schematically in accordance with different embodiments of the present disclosure.

### DETAILED DESCRIPTION OF ACCOMPANYING DRAWINGS

Prior to describing the invention in detail, definitions of certain words or phrases used throughout this patent document will be defined: the terms "include" and "comprise", as well as derivatives thereof, mean inclusion without limitation; the term "or" is inclusive, meaning and/or; the phrases "coupled with" and "associated therewith", as well as derivatives thereof, may mean to include, be included within, interconnect with, contain, be contained within, connect to or with, couple to or with, be communicable with, cooperate with, interleave, juxtapose, be proximate to, be bound to or with, have a property of, or the like; Definitions of certain words and phrases are provided throughout this patent document, and those of ordinary skill in the art will understand that such definitions apply in many, if not most, instances to prior as well as future uses of such defined words and phrases.

Reference throughout this specification to "one embodiment," "an embodiment," or similar language means that a particular feature, structure, or characteristic described in connection with the embodiment is included in at least one embodiment. Thus, appearances of the phrases "in one embodiment," "in an embodiment," and similar language throughout this specification may, but do not necessarily, all refer to the same embodiment, but mean "one or more but not all embodiments" unless expressly specified otherwise. The terms "including," "comprising," "having," and variations thereof mean "including but not limited to" unless expressly specified otherwise. An enumerated listing of items does not imply that any or all of the items are mutually exclusive and/or mutually inclusive, unless expressly specified otherwise. The terms "a," "an," and "the" also refer to "one or more" unless expressly specified otherwise.

Although the operations of the disclosed method (not claimed) may be described in a particular, sequential order for convenient presentation, it should be understood that the disclosed methods can encompass an order of operations other than the particular, sequential order disclosed. For example, operations described sequentially may in some cases be rearranged or performed concurrently. Further, descriptions and disclosures provided in association with one particular method are not limited to that method, and may be applied to any method disclosed herein. Moreover, for the sake of simplicity, the attached figures may not show the various ways in which the disclosed system, method, and apparatus can be used in combination with other systems, methods, and apparatuses.

Furthermore, the described features, advantages, and characteristics of the embodiments may be combined in any suitable manner. One skilled in the relevant art will recognize that the embodiments may be practiced without one or more of the specific features or advantages of a particular embodiment. In other instances, additional features and advantages may be recognized in certain embodiments that may not be present in all embodiments. These features and advantages of the embodiments will become more fully apparent from the following description and apportioned claims, or may be learned by the practice of embodiments as set forth hereinafter.

It should be noted that in the figures and the description to follow, the terms "frame" or "stent" or "frame" or "scaffold structure" or "support frame" or "scaffold" refer to the metallic frame of this invention. These terms are used interchangeably but carry the same meaning. The term "valve" or "prosthetic valve" refer to the prosthetic valve of the present invention assembled prosthetic valve using a support frame and other components like leaflets of animal tissue, skirt, etc. These terms are also used interchangeably. The term "native valve" is used for the natural valve in human heart.

Likewise, the terms 'delivery system', 'delivery catheter', 'catheter', 'balloon catheter', 'delivery balloon catheter' refer to the delivery apparatus used in the present invention. These terms are used interchangeably but carry the same meaning.

The present disclosure describes a balloon expandable prosthetic heart valve system (or system). The system of the present invention includes a trans-catheter prosthetic heart valve (THV) and a THV delivery system. The THV of the present invention may be implanted via a catheterization technique in a human stenosed aortic orifice using the THV delivery system. The THV and the THV delivery system work in unison to achieve an improved performance of the system.

The present invention ingeniously retains the core legacy technology of the THV disclosed in the aforementioned patent application provided in the background with several enhancements incorporated. The THV includes a flexible frame which can expand and collapse, a plurality of leaflets (preferentially three leaflets) formed from animal tissue or synthetic material, an internal skirt and an external skirt that are attached to the frame.

The frame of the THV in the present invention offers several structural and clinical advantages over the conventional frames and mitigates the disadvantages offered by the same. The frame of the THV of the present invention includes interlaced octagonal cells which incorporate a rhombus body (with/without holes) or a diamond shaped cell at each intersection. Such a structure enhances columnar strength thereby resulting in improved radial strength and fatigue resistance.

The frame includes two rows of tessellating octagonal cells placed one above the other as opposed to a traditional trans-catheter prosthetic valve having a frame with two rows of cells. The reduction in number of rows and the specific shape of the cells results in reduced foreshortening of the frame on radial expansion which makes it easier for the operator to implant the prosthetic heart valve accurately.

Further, the delivery system helps in accurate placement and precise deployment of the THV of the present invention.

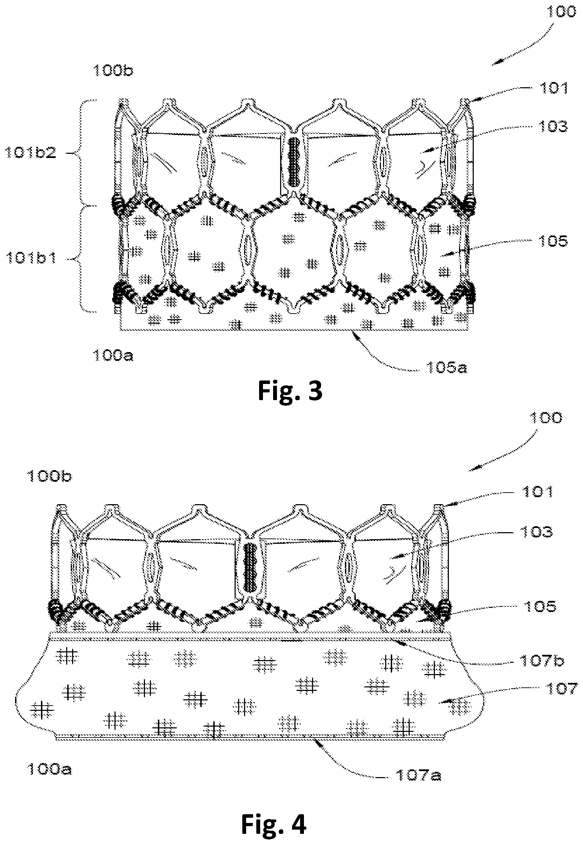

The THV 100 in accordance with an embodiment of the present invention is represented in FIGs. 3 & 4. Fig. 3 shows THV 100 without external skirt 107, while Fig. 4 shows THV 100 with external skirt 107. As the THV 100 is implanted in a human stenosed aortic orifice, the THV 100 may also be referred as a 'prosthetic aortic valve'.

The frame (also referred to as "support frame") of THV 100 is radially expandable and radially collapsible. The THV 100 is suitable for mounting on a balloon of a delivery catheter in a radially collapsed condition. The balloon delivery catheter along with THV 100 in collapsed condition is navigated to the implantation site where THV 100 is implanted in the human stenosed aortic orifice by radially expanding THV 100. The THV 100 exhibits fluoroscopic properties.

The THV 100 includes an inflow end 100a and an outflow end 100b. Blood enters the THV 100 at the inflow end 100a and leaves at the outflow end 100b.

As shown in FIGs. 3 & 4, the THV 100 includes a frame 101 (or support frame 101), a plurality of leaflets 103, an internal skirt 105 (as shown in FIG. 3) and an external skirt 107 (as shown in FIG. 4).

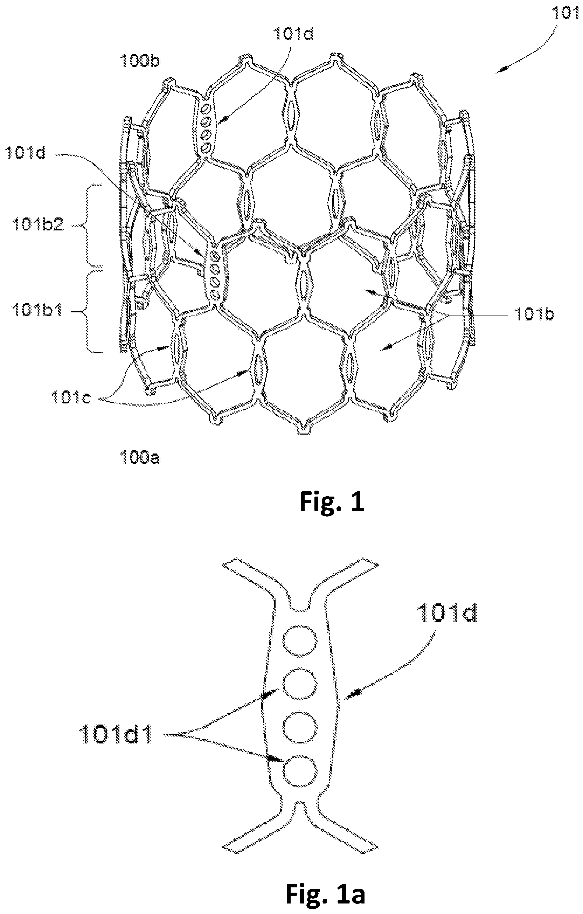

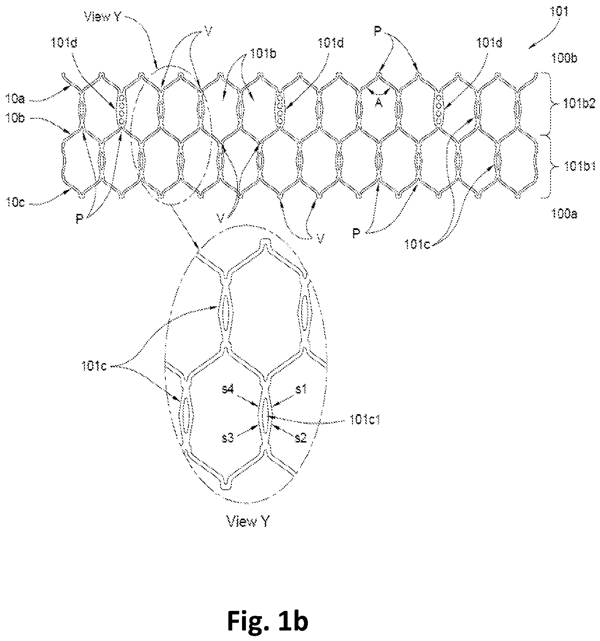

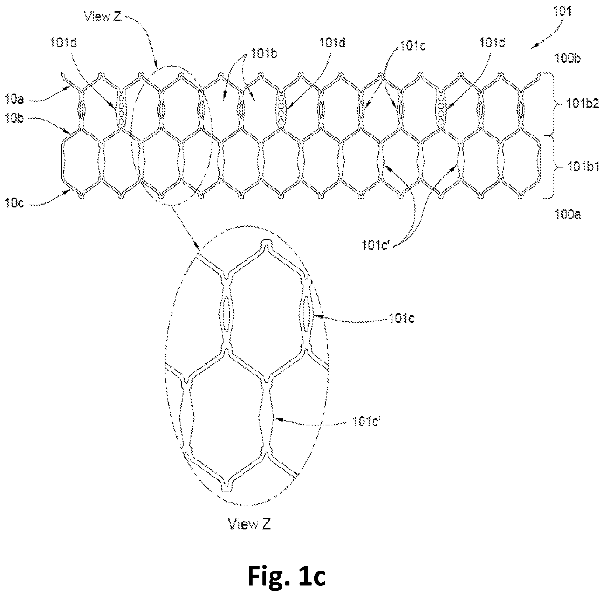

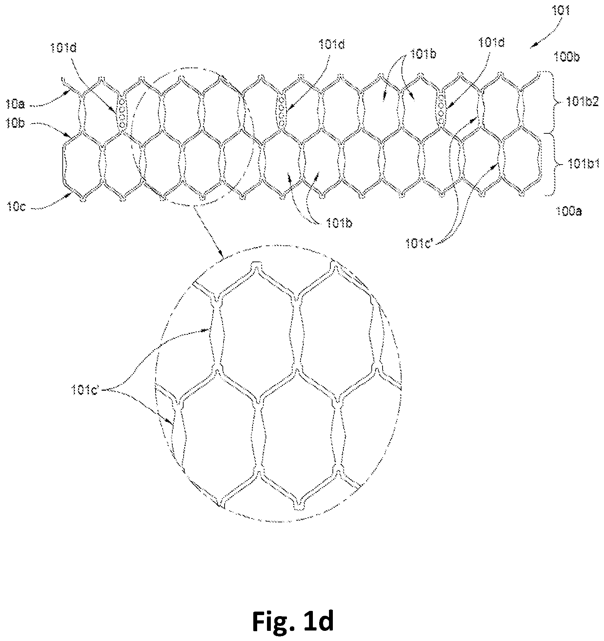

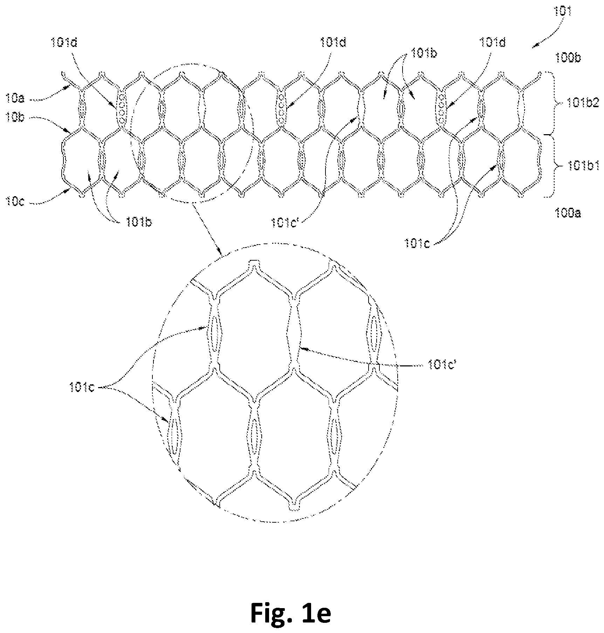

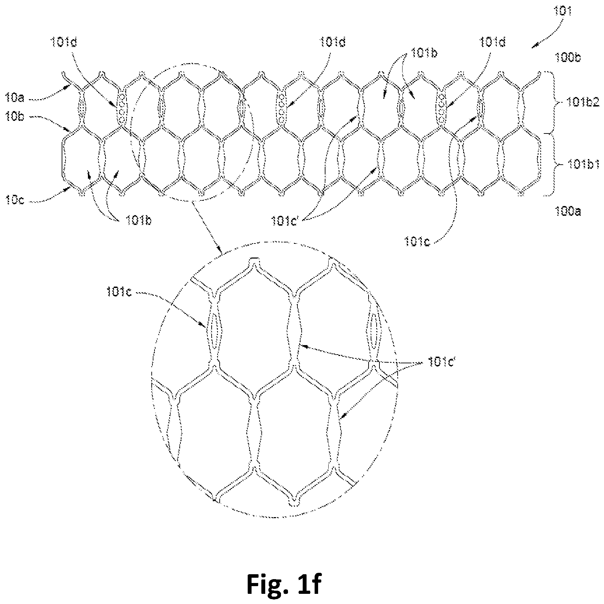

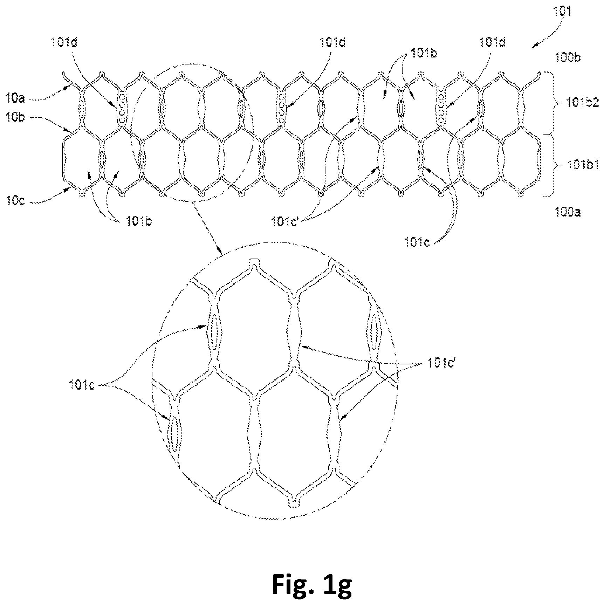

The exploded views of two exemplary embodiments of the frame 101 of the present invention are shown in FIGs. 1, 1b and 1c. The frame 101 is a radially collapsible and radially expandable frame having a cylindrical shape with an inflow end 100a and an outflow end 100b. The frame 101 of the exemplary embodiment is a balloon-expandable frame. Alternately, the frame 101 may be a self-expandable frame.

The frame 101 may be formed by following a pre-defined methodology. For example, the frame of THV 100 may be formed by laser cutting a metal tube. The metal tube may be made from a metal or a metal alloy, including but not limited to, stainless steel, cobalt-chromium alloy, cobalt-chromium-nickel alloy, cobalt-chromium-nickel-molybdenum alloy such as MP35N, Nitinol etc. The material used for the frame 101 may be fluoroscopic. In a preferred embodiment of the present invention, the frame 101 is balloon expandable and is made from a tube of cobalt-chromium-nickel-molybdenum alloy viz. MP35N which ensures optimal radial strength, radiopacity and prompt MRI compatibility of THV frame 101.

In an embodiment, a titanium niobium nitride (TiNbN) ceramic surface coating may be provided at least on the outer surface of the frame 101. This coating has high biocompatibility and offers following benefits.
1. Enhanced radial strength of the frame.
2. Enhanced radiopacity of the frame
3. Improved fatigue resistance of the frame.
4. Reduced allergic reaction caused by cobalt and other components of the alloy used for the frame.
5. Very low roughness average (RA value) due to high surface finish offered by TiNbN coating which minimizes the propensity for platelet aggregation or any potential thrombus formation or pannus formation.

The structure of the frame 101 of an exemplary embodiment is shown in Figs. 1, 1a and 1b. It should be noted in relation to Fig. 1b that the frame scaffold design is shown laid flat for convenience purpose only and the frame 101 may not be created from a flat metal sheet.

As evident from the FIGs. 1 and 1b, the frame 101 includes an inflow end 100a, an outflow end 100b and a plurality of rows of cells 101b. The cells 101b include a pre-defined octagonal shape. In an embodiment as shown in FIGs. 1 and 1b, the frame 101 includes two rows i.e. a lower row of cells 101b1 (towards the inflow end 100a) and an upper row of cells 101b2 (towards the outflow end 100b) of tessellating octagonal cells 101b placed one above the other (adjacently placed) extending between its distal end and the proximal end.

Referring also to Figs. 3 and 4, the blood enters the THV 100 at the inflow end 100a (also referred to as "lower end" or "distal end") and leaves at the outflow end 100b (also referred to as "upper end" or "proximal end"). The row of octagonal cells 101b at the inflow end 100a/distal end of the frame 101 is also referred to as the lower row of cells 101b1. The row of octagonal cells 101b at the outflow end 100b/proximal end of the frame 101 is also referred to as the upper row of cells 101b2.

The embodiment as shown in FIGs. 1 and 1b has two rows of cells which is less than three rows of cells in the aforesaid patent application. The reduction in the number of rows and the specific shape of the cells 101b result in reduced foreshortening of the frame 101 on radial expansion which makes it easier for the operator to implant the THV 100 accurately. Further, the use of octagonal cells 101b offer high columnar and radial support to the structure.

The frame 101 as shown in FIGs. 1 and 1b is homogeneous i.e. the cells 101b in the upper row 101b2 and the lower row 101b1 are of same or equal size. However, the frame 101 having cells 101b of different sizes is also within the scope of the present invention. For example, the size of the cells 101b of the frame 101 in the upper row 101b2 may be larger or smaller than the size of the cells 101b in the lower row 101b1.

The sum of the angles formed by a polygon is (n-2)*180° where n denotes the number of sides. Hence, for an octagon (with 8 sides), the sum of the angles would be 1080°. Accordingly, sum of all angles of any of the octagonal cells 101b of the frame 101 of the exemplary embodiment is 1080°.

As evident from FIGs. 1b, the said rows of cells 101b1 and 101b2 of the frame 101 are formed by three circumferentially extending rows of angled struts (also referred to as simply 'angled struts') 10a, 10b, 10c. The circumferentially extending rows of angled struts_include a first row of angled struts 10a (or upper row of angled struts 10a), a second row of angled struts 10b (or middle row of angled struts 10b) and a third row of angled struts 10c (or lower row of angled struts 10c). The first row of angled struts 10a is disposed at the proximal end 100b of the frame 101. The third row of angled struts 10c is disposed at the distal end 100a (or inflow end) of the frame 101. The second row of angled struts 10b is disposed between the first row of angled struts 10a and the third row of angled struts 10c.

In an embodiment, the angle ('A') between two angled struts in the depicted embodiments of FIG. 1b is 116°. However, it should be noted that the said angle may be less than or more than 116°.

Each of the circumferentially extending rows of angled struts 10a, 10b, 10c may include an undulating shape with a plurality of peaks 'P' and valleys 'V' as shown in Fig. 1b. Hence, any two consecutive angled struts of a circumferentially extending row of angled struts 10a/10b/10c either form a peak P or a valley V. In an exemplary embodiment as shown in FIG. 1b, the peaks 'P' of the first row of angled struts 10a face the valleys 'V' of the second row of angled struts 10b. Likewise, the peaks 'P' of the second row of angled struts face the valleys 'V' of the third row of angled struts 10c. A peak of one row of angled struts facing a valley of an adjacent row of angle struts is termed as corresponding peak or valley.

The above defined circumferentially extending rows of angled struts 10a, 10b and 10c are connected to each other by links to form the adjacently placed first and the second rows of cells 101b1 and 101b2 of the frame 101. Each link is either a diamond shaped cell 101c or a solid rhombus body with holes (101d). There are three rhombus bodies (101d) in the upper row of cells 101b2.

In the embodiment of the frame structure shown in Fig. 1b, the upper row of cells 101b2 is formed by connecting the valleys V of the upper row of angled struts 10a with the corresponding peaks P of the middle row of angled struts 10b by links. Each link is either a diamond shaped cell defined by a pair of crooked struts or solid rhombus body. The pair of crooked struts (s1/s2 and s3/s4 as shown in exploded view Y) form diamond shaped cells 101c. Therefore, the upper row of cells 101b2 includes interlaced octagonal cells creating alternate sequence of rhombus bodies or diamond shaped cells at each junction. There are three rhombus bodies 101d with four holes spaced uniformly at 120° with respect to each other. Fig. 1a shows the details of an exemplary rhombus body 101d with four holes101d' which are provided for suturing the commissural tabs of the leaflets. It may be noted that the number of holes may be less than or more than four. In this embodiment, the lower row of cells 101b1 is formed by connecting the valleys V of the middle row of angled struts 10b with the corresponding peaks P of the lower row of angled struts 10c by links. Each link is a diamond shaped cell defined by a pair of crooked struts (s1/s2 and s3/s4 as shown in exploded view Y) which form diamond shaped cells 101c. The interconnection of the one row and the adjacent row of angled struts 10a/10b/10c via such links results in a cell structure having interlaced octagonal cells 101b and diamond shaped cells 101c or solid rhombus bodies 101d with holes.

It should be noted in relation to Fig. 1c that the frame scaffold design is shown laid flat for convenience purpose only and the frame 101 may not be created from a flat metal sheet. In this embodiment, the upper row of cells 101b2 is formed by connecting the valleys 'V' of the first row of angled struts 10a to the corresponding peaks 'P' of the second row of angled struts 10b by links. Each link is defined by a diamond shaped cells formed by a pair of crooked struts or solid rhombus bodies. Therefore, the upper row of cells 101b2 includes interlaced octagonal cells creating alternate sequence of rhombus bodies or diamond shaped cells at each junction. The upper row of cells 101b2 in this embodiment is similar to the upper row of cells in the embodiment of Fig. 1b where the links consist of three rhombus bodies 101d with holes and the rest are diamond shaped cells 101c. In this embodiment, the lower row of cells 101b1 is formed by connecting the valleys 'V' of the second row of angled struts 10b to the corresponding peaks 'P' of the third row of angled struts 10c by rhombus bodies 101c' without holes. View Z shows a blown-up view of the links 101c and 101c'.

The exemplary structures described above help to enhance columnar strength of the frame 101 resulting in improved radial strength and fatigue resistance of the frame 101. The details of interlaced octagon are shown in zoomed view Y of a portion of the frame 101 in Fig. 1b and zoomed view Z of a portion of the frame 101 in Fig. 1c.

As shown in Figs. 1b-1c, the diamond shaped cells 101c have an open configuration; i.e. any diamond shaped cell 101c includes an opening 101c1 enclosed within a pair of crooked struts s1/s2 and s3/s4. The diamond shaped cell 101c with the opening 101c1 is also referred to as "open rhombus body" (rhombus body with open structure). The solid rhombus body is a diamond shaped cell with no opening (rhombus body with solid structure).

A skilled person could and would think of a number of alternate frame scaffold structures with various combinations of diamond shaped cells (101c) and rhombus bodies (101c'/101d) linking the angled struts 10a/10b/10c. A few exemplary embodiments of frame scaffold structures are described below. In all the embodiments described below, the frame scaffold design is shown laid flat for convenience purpose only and the frame 101 may not be created from a flat metal sheet.

In another exemplary embodiment shown in Fig. 1d, the upper row of cells 101b2 are formed by connecting the valleys V of the upper row of angled struts 10a with the corresponding peaks P of the middle row of angled struts 10b by links. A link is defined by rhombus bodies 101c' (without holes) or 101d (with holes). Therefore, the upper row of cells 101b2 includes interlaced octagonal cells creating alternate sequence of rhombus bodies (with or without holes) at each junction. There are three rhombus bodies 101d with four holes spaced uniformly at 120° with respect to each other. The rest of the links are rhombus bodies 101c' without holes. In this embodiment, the lower row of cells 101b1 is formed by connecting the valleys V of the middle row of angled struts 10b with the corresponding peaks P of the lower row of angled struts 10c by links formed by rhombus bodies without holes (101d).

Other exemplary embodiments of the frame scaffold structure are shown in Figs. 1e-1g where the links joining the rows of angled struts with different combinations of diamond shaped cells 101c, rhombus bodies 101c' without holes and rhombus bodies 101d with holes.

For example, the frame structure of the embodiment of Fig. 1e where the cells of the upper row 101b2 are formed with links 101c, 101c' and 101d, while the cells of the lower row 101b1 are formed with links 101c.

The frame structure of another exemplary embodiment is shown in Fig. 1f where the cells of the upper row 101b2 are formed with links 101c, 101c' and 101d, while the cells of the lower row 101b1 are formed with links 101c'.

Another exemplary embodiment of a frame structure is shown in Fig. 1g where the cells of the upper row 101b2 are formed with links 101c, 101c' and 101d, while the cells of the lower row 101b1 are formed with links 101c and 101c'.

There are several other alternative configurations of providing diamond shaped cells 101c and rhombus bodies 101d/101c' (with or without holes) readily realised by a skilled person in light of the above disclosure and covered within the teachings of the present invention.

In all the embodiments described above, the upper row of cells 101b2 includes three rhombus bodies 101d with holes, angularly located at 120° with respect to each other. These rhombus bodies 101d with holes form commissure attachment areas which have a plurality of holes 101d1 (as shown in FIG. 1a) for suturing commissures of the leaflets 103 to the frame 101 (as described below). In a preferred embodiment shown in Fig. 1a, each commissure attachment area 101d includes four holes 101d1. However, it should be noted that the number of holes 101d1 can be more than or less than four.

In an embodiment, at least one radiopaque marker (not shown) may be provided on the frame 101 on any of the struts preferably on the crooked struts forming the diamond shaped cells 101c or on rhombus bodies 101c' for easy visualization under fluoroscopy. In a preferred embodiment, the at least one radiopaque marker is provided on the struts of diamond shaped cells 101c or on rhombus body 101c' located in the lower row of cells 101b1. In another preferred embodiment, the at least one radiopaque marker may be provided on at least one rhombus body 101c'.

It is obvious that the rhombus body 101c' or 101d with solid structure, has more metal than the diamond shaped cell 101c with open structure. Hence, the rhombus body 101c'/101d will exhibit higher radiopacity than the diamond shaped cells 101c. The higher radiopacity is helpful in accurate placement of the THV 100 due to better visualization under fluoroscopy as described further.

The THV 100 of the present invention further includes a plurality of leaflets. In an embodiment, the THV 100 includes three leaflets. The leaflets may be made from any bio-compatible material with sufficient flexibility to allow movement of leaflets. For example, in the present invention, the leaflets of a preferred embodiment are made from an animal tissue such as bovine pericardial tissue. Alternately, the leaflets may be formed from a synthetic polymeric material.

The skilled person is aware of the function of the leaflets of a prosthetic heart valve which allows unidirectional flow of blood from inflow end 100a of THV 100 to the outflow end 100b and prevents the flow of blood in reverse direction. This is achieved by opening and closing the leaflets during systolic and diastolic cycles.

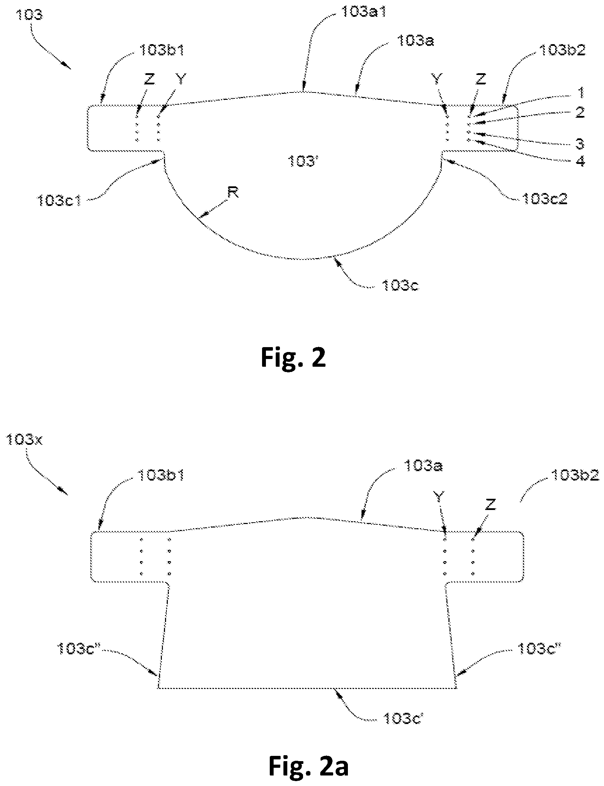

An exemplary embodiment of the structure of the leaflet 103 is shown in FIG. 2. As depicted, each leaflet 103 of this embodiment may include a body 103' having a relatively straight upper edge 103a. The embodiment shown in FIG. 2 has an apex 103a1. However, alternately, the apex 103a1 may be absent. The upper edge 103a is kept free for coaptation with the corresponding free edges of the other leaflets 103.

The upper edge 103a of each leaflet 103 may extend into oppositely disposed side tabs (or commissure tabs) marked as 103b1, 103b2 at either side of the leaflet 103. A plurality of holes may be disposed at both the side tabs 103b1, 103b2 for ease of suturing. In the embodiment shown in FIG. 2, two vertical rows of holes marked as Y, Z are provided near the portion of each side tab 103b1, 103b2 near the body 103' of the leaflet 103. In an embodiment, each row of holes Y, Z may include four holes (marked as 1, 2, 3, 4). The number of holes may be more than or less than four. Similarly, the number of vertical rows of holes may be one or more than two. The said holes are utilized for attachment of the leaflets 103 to the connecting fabric and/or frame 101 by suturing.

Each leaflet 103 may further include a lower edge 103c. As shown in the embodiment of Fig. 2, the lower edge 103c may include a scalloped shape with optional small straight portions 103c1 and 103c2 located at the junction of the lower edge103c and the side tabs 103b1, 103b2. The scalloped lower edge of the leaflet 103 of a preferred embodiment may include a constant radius R. However, the scalloped lower edge of the leaflet 103 may have varying radius. The scalloped lower edge of each of the leaflets 103 may be attached to the internal skirt 105 by any known method such as suturing.

Alternately, the THV 100 may include a leaflet 103x as shown in embodiment of FIG. 2a, which is similar to the embodiment shown in FIG. 2, except that it has a straight lower edge 103c' and the sides 103c" which are vertically oriented (unlike scalloped lower edge) as in the embodiment of FIG. 2. The sides 103c" may be vertical or at an angle to the bottom straight edge 103c'. Fig. 2a shows an embodiment where the sides 103c" are not exactly vertical but are at an angle with respect to bottom edge 103'.

The above defined leaflets 103/103x may be attached to the frame 101 using a pre-defined method. A skilled person is well aware of various methods known in the art of attaching leaflet tabs to the commissure areas 101d of the frame 101 using one or more supporting fabrics. One of the side tabs 103b1/103b2 of a given leaflet 103 or 103x is paired with one side tab 103b1/103b2 of another leaflet 103 or 103x to form a leaflet-commissure. The leaflet-commissures may then be attached to the commissure areas 101d of the frame 101 using supporting fabric so as to avoid direct contact of the tissue with metal of the frame 101. As described above, the lower edge 103c of leaflet 103 (FIG. 2) may be attached to the internal skirt 105. Similarly, the straight lower edge 103c' and vertically oriented edges 103c" of leaflet 103x (Fig. 2a) may also be attached to the internal skirt 105.

The internal skirt 105 is attached to the inner (or internal) surface of the frame 101 and in a preferred embodiment, covers the internal surface of the lower row 101b1 of the octagonal cells 101b at least partially as shown in FIG. 3. The scalloped lower edge 103c or the straight lower edge 103c'of the leaflets 103 or 103x is attached to the inner surface of the internal skirt 105. The vertically oriented edges 103c" of the leaflets 103x are also attached to the inner surface of the internal skirt 105. The internal skirt 105 of a preferred embodiment may be made from a fabric such as PET. However, any other biocompatible fabric or animal tissue with required flexibility, strength and porosity can be used for making the internal skirt 105. The internal skirt 105 prevents leakage of blood from the openings of the cells 101b of the frame 101 in the lower row 101b1 and also prevents inadvertent damage of the leaflets 103/103x of THV 100 by calcium spicules present in diseased native valve.

The external skirt 107 of an exemplary embodiment is shown in Fig. 4. The external skirt 107 includes an upper end 107b and a lower end 107a. In an embodiment, the upper end 107b (towards the outflow end) of the external skirt 107 may be attached to the internal skirt 105 and the frame 101 via suturing at an intermediate portion of the frame 101 as shown in Fig. 4. In the same embodiment, the lower end 107a (towards the inflow end 100a) of the external skirt 107 is attached to the lower end 105a of the internal skirt 105 by suturing. As known to a skilled person, the function of the external skirt 107 is to plug microchannels between THV 100 and the inner surface of the vasculature and prevent or minimize paravalvular leakage.

Fig. 4 shows an exemplary embodiment in which the external skirt 107 is attached to the outer (or external) surface of the frame 101 and it covers the external surface of the lower row 101b1 of the octagonal cells 101b at least partially. The extent of this covering further assists in placing the THV 100 with minimal error across asymmetric cusp geometry, cusp with severe calcification, anatomically challenging aorta like horizontal aorta and reduces the operator learning curve during placement and deployment.

The external skirt 107 of the present invention may be made from a fabric such as PET. However, any other biocompatible fabric or material like animal tissue with required flexibility, strength and porosity can be used. Further, as shown in Fig. 4, the external skirt 107 has excess material which fits loose onto the frame 101 forming a slack when the frame 101 is in radially expanded condition. The excess material of the loose external skirt 107 fills the irregular inner surface of the aortic annulus (which also has native leaflets) and plugs micro channels and thus preventing or minimizing the paravalvular leakage. The slack reduces when the frame 101 is in a radially collapsed condition.

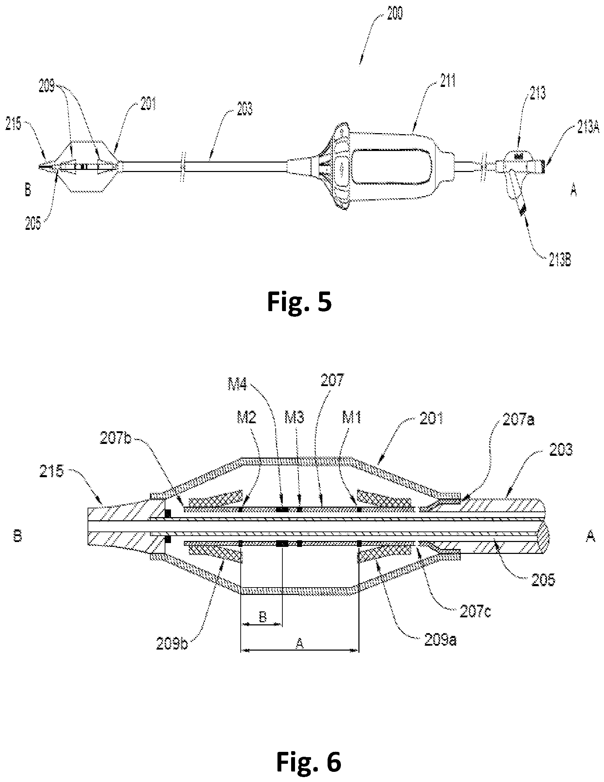

The delivery system i.e. a delivery catheter 200 for the THV 100 of this invention will now be described. Fig. 5 depicts an exemplary delivery catheter 200. The delivery catheter 200 is utilized for deploying the THV 100 within a diseased native valve at a target location. The frame structure of THV 100 and the delivery catheter 200 of the instant invention provide an easy and accurate method for deployment of the THV 100 at the target location.

The delivery catheter 200 as shown in FIG. 5 is a balloon catheter. A skilled person is well aware of the construction of a balloon catheter used for radially expanding a balloon expandable device such as a stent or a prosthetic valve. The exemplary delivery catheter 200 includes a proximal end A and a distal end B. The delivery catheter 200 further includes a balloon 201 at its distal end B (shown in Fig. 6), an outer shaft 203, an inner shaft 205, a support tube 207, one or more stoppers 209, a handle 211 and a connector 213 at the proximal end.

The outer shaft 203 is in the form of an elongated external tube referred also as 'elongated shaft'. The outer shaft 203 defines an outer lumen through which the inner shaft 205 extends coaxially. The inner shaft 205 defines an inner lumen. A guidewire passes through the inner lumen.

The outer shaft 203 and the inner shaft 205 have respective proximal and distal ends (A and B respectively). Proximal end is towards the handle 211 i.e. towards the operator. The opposite end towards the balloon 201 is the distal end which is away from the operator. The proximal ends of the outer shaft 203 and the inner shaft 205 may pass through the handle 211 and may be attached to the connector 213. The connector 213 may be a Y-shaped connector having a port 213A for exit of a guidewire and a port 213B for injecting inflation fluid into the catheter 200. The guidewire port 213A is in communication with the inner lumen. The port 213B for inflation fluid is in communication with the annular space between the two shafts 203 and 205. A skilled person would appreciate that this arrangement is normally provided in a balloon catheter.

An exemplary embodiment of the balloon 201 is shown in Fig. 6. The balloon 201 is attached to the distal end of the outer shaft 203. The inner lumen 205 extends through the balloon 201 and it ends into a soft tip 215 at a distal most end of the catheter 200. The guidewire (not shown) enters the guidewire lumen at the distal end of the soft tip 215 of the catheter 200, passes through the inner lumen, passes through the balloon 201 and exits from the connector 213 at guide wire port 213a.

The balloon 201 is an inflatable balloon that is radially expanded by injecting pressurised inflation fluid into the balloon 201 through the annular space between the outer shaft 203 and the inner shaft 205.

In a preferred embodiment, a support tube 207 is attached to the distal end of the outer shaft 203. The support tube 207 extends within the balloon 201 and the inner shaft 205 passes through the support tube 207 coaxially as more clearly shown in the FIG. 6.

As shown in FIG. 6, the support tube 207 includes a proximal end 207a and a distal end 207b. The proximal end 207a is attached to the outer shaft 203. The distal end 207b is a free end and overhangs within the balloon 201.

The delivery catheter 200 may include at least one stopper made from a resilient and biocompatible material. A preferred embodiment of Fig. 6 is provided with two stoppers; a proximal stopper 209a and a distal stopper 209b. As shown in FIG. 6, the proximal and distal stoppers 209a, 209b are attached to the outer surface of the support tube 207.

The proximal stopper 209a and the distal stopper 209b may be spaced apart at a pre-defined distance. In the preferred embodiment, the clear gap between the distal end of the proximal stopper 209a and the proximal end of the distal stopper 209b is little more than the length of the crimped THV 100. The THV 100 is crimped on the balloon 201 within this gap. The clear gap as defined above may vary depending upon the length of the crimped THV 100.

Crimping THV 100 between the proximal stopper 209a and the distal stopper 209b as described above, prevents the THV 100 from shifting on or dislodging from the balloon 201 during insertion of the crimped THV 100 into the patient's vasculature and while manoeuvring the THV 100 through tortuous vascular pathway to reach implantation site. The stoppers 209a and 209b also prevent inadvertent valve embolization during balloon inflation. The stoppers 209a and 209b create a lower entry profile at their respective ends due to their resilient nature which assists the smooth exit of the THV 100 from an introducer sheath into the patient's aorta (vasculature) and also for easy retrieval of an undeployed THV 100. The inflation fluid enters into the balloon 201 through holes 207c in the support tube 207 at its proximal end 207a and also from its free and open distal end 207b. This feature ensures steady expansion of the balloon 201 simultaneously from the distal and proximal end creating a dog bone which stabilizes the THV 100 during expansion and prevents inadvertent valve embolization.

A skilled person will readily realize that the support tube 207 described above is to ease the accurate attachment of the stoppers 209a, 209b and for providing free passage to inflation fluid into the balloon 201. A delivery system without the support tube 207 would also function. In this case, the stoppers may be located on the inner shaft 205.

The support tube 207 of the present invention may include a plurality of radiopaque marker bands (or markers). In a preferred embodiment, the support tube 207 includes four radiopaque marker bands including a proximal marker band M1, a distal marker band M2, a middle marker band M3 and a landing zone marker band M4. If the delivery system 200 does not have a support tube 207, these markers may be provided on the inner shaft 205 on the portion located within the balloon 201.

The above description refers to a specific THV frame scaffold structure 101, which has octagonal cells 101b with interlaced diamond shaped cells 101c or rhombus bodies 101c' and commissure areas 101d of specific shape. A skilled person would readily realize that the concept of providing radiopaque markers (viz. proximal marker, distal marker, middle marker and landing zone marker as disclosed below) for accurate placement of the THV 100 at orthotopic position can be applied to a frame scaffold structure with cells of any polygonal shape (e.g. diamond shape, hexagonal shape etc.).

As the name suggests, the proximal marker band M1 and the distal marker band M2 are disposed towards the proximal and distal ends 207a, 207b respectively of the support tube 207. The middle marker band M3 is located between the proximal and distal marker bands M1, M2 equidistant from M1 and M2. In an embodiment, the landing zone marker M4 is placed between the distal and mid marker bands M2, M3 at a distance of around 32-33% of the distance between proximal and distal markers M1, M2 from the distal end marker M2 i.e. dimension B is 32-33% of dimension A as shown in FIG. 6.

The landing zone marker M4 plays a guiding role in accurate positioning of the THV 100 at the implantation site to achieve implantation at the most preferred location viz. orthotopic position. Accurate positioning of THV 100 could be achieved without landing zone marker M4 as described below.

The distal end of the shaft of the exemplary catheter 200 may be configured to be flexed in controlled manner for easy passage through aortic arc. In an alternate embodiment, the distal end of the shaft of catheter 200 may not be configured to be flexed. In yet another alternate embodiment, the distal end of the shaft of the catheter 200 may be pre-shaped with a fixed radius for easy passage through aortic arc. The pre-shaping of the catheter shaft can be achieved by known methods such as thermal treatment.

The following description is related to the replacement of diseased aortic valve.

Accurate placement and precise deployment of a prosthetic valve in aorta is very important to achieve optimal performance i.e. reduced valve gradients (sustained hemodynamics), absence of paravalvular regurgitation and avoiding any iatrogenic damage to conduction system that necessitates a new permanent pacemaker implantation. The ideal location of implantation of a prosthetic valve in aorta is preferably the orthotopic position where the attempt is to superimpose the prosthetic annulus (neo-annulus) to the native annulus ring. Implanting the prosthetic valve at this location has three important advantages as outlined below.

One advantage of placement of prosthetic valve in orthotopic position is better anatomical placement of the prosthetic valve. The annulus of the diseased native valve and its leaflets are stenosed and may also be calcified. When a prosthetic valve, sized to match the native annulus is expanded at the orthotopic position, the frame is held firmly within the stenosed annulus which may have calcified leaflets, thereby offering geographical fix which eliminates risk of embolization of the prosthetic valve by dislodgement.

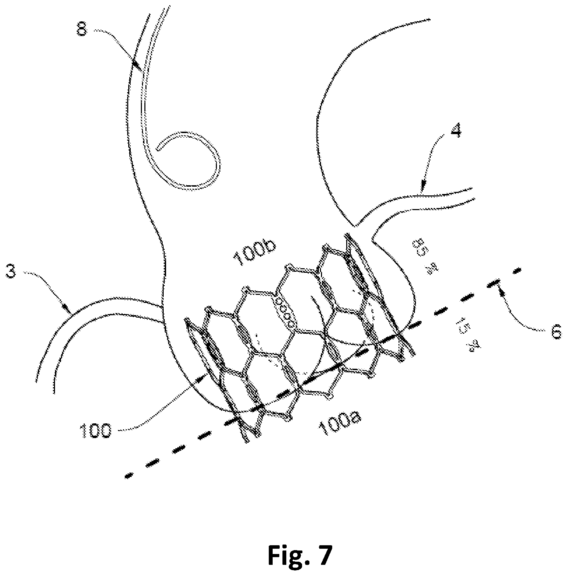

The second advantage of placement of prosthetic valve in orthotopic position is minimal protrusion of valve in left ventricle. It is important to deploy prosthetic valve at its annular position and not too deep towards ventricular end, also known as infra-annular position due to two important sub-aortic anatomical zones. First zone is the membranous septum which has densely populated cardiac conduction musculature atrioventricular node (AV node) which transfers electrical impulses to maintain normal heart rhythm. It is important that the prosthetic valve does not dwell into the left ventricle outflow tract (LVOT) thereby not disturbing cardiac conduction system. The second zone is the aortomitral curtain and position of native mitral valve which is located postero-laterally to the aortic valve. Wrongly positioned prosthetic valve may have the propensity to interfere with normal functioning of the anterior leaflet of the mitral valve, thus impacting the functioning of the mitral valve. The THV 100 implanted by the method recommended herein achieves minimum protrusion of the prosthetic valve in LVOT. Fig. 7 shows depicts the aortic root complex (described in detail below) schematically. In an embodiment, as shown in Fig. 7, 80%-85% of the length of the expanded THV 100 remains above aortic annulus while, the residual 15%-20% of the length of the expanded THV 100 dwells in the sub-valvular space i.e. virtual annular plane 6.

The third advantage of placement of prosthetic valve in orthotopic position is minimizing obstruction to ostia of coronary arteries (shown as 3 and 4 in Fig. 7) which are located along the coronary sinus of valsalva or may be above the sino-tubular junction. Ideally, the prosthetic valve should not obstruct the blood flow into these coronary arteries by obstructing or causing 'jailing' of their ostia. The precise location of the THV 100 at orthotopic position prevents this by minimizing the protrusion of the frame 101 into the ascending aorta. The jailing of the ostia of coronary arteries (3 and 4) is further avoided in the present invention by large uncovered cells at the outflow end and the short frame height of the expanded THV 100.

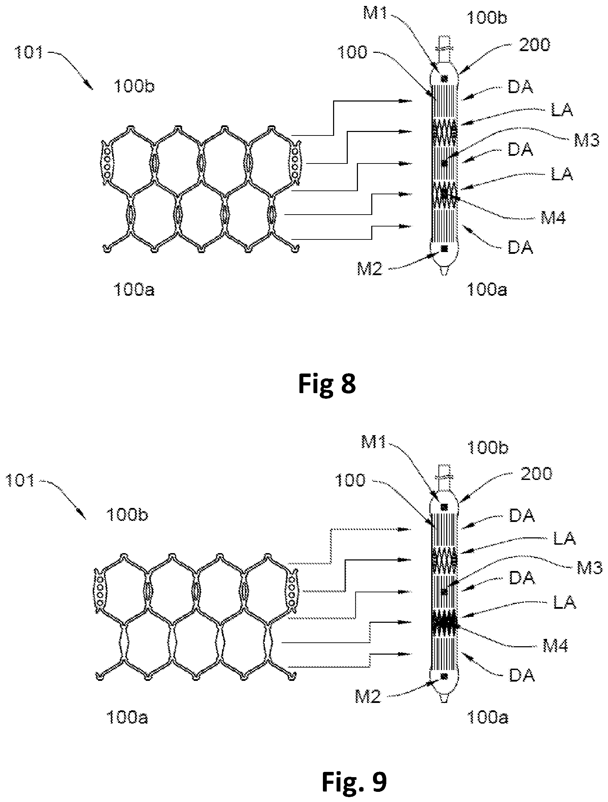

As shown in Figs. 1b-g, the frame 101 of the THV 100 has three circumferentially extending rows of angled struts 10a, 10b, 10c that are interconnected by diamond shaped cells 101c, rhombus bodies 101c' and the commissure areas 101d. The THV 100 is crimped on the balloon 201 between the two stoppers 209a, 209b and two extreme radiopaque markers M1, M2. Under fluoroscopy, the frame 101 of the THV 100 as depicted in FIGs. 1 and 1b, under collapsed condition on the balloon 201, exhibits alternate light areas (LA) and dense areas (DA) as shown schematically in Fig. 8. The dense areas DA are formed by circumferentially extending rows of angled struts (10a, 10b, 10c) and light areas LA are formed by the crooked struts (s1/s2/s3/s4) of the diamond shaped cells 101c and the commissure areas 101d which interlace the octagonal cells 101b. When the THV 100 is crimped on the balloon 201 of the delivery system 200 between the two stoppers 209a, 209b and extreme radiopaque markers M1, M2, the landing zone marker M4 is found behind the mid-point of the first light area towards the inflow end 100a of the THV 100 as shown schematically in Fig. 8 which depicts the embodiment of frame 101 and Fig. 101b.

In the embodiment of FIG. 1c, the lower row 101b1 of cells 101b includes rhombus bodies 101c', which offer relatively higher radiopacity compared to that offered by diamond shaped cells 101c. Hence, the light area (LA) towards the inflow end 100a of the crimped frame 101 will be relatively darker compared to the LA formed by the diamond shaped cells 101c and would be easier to identify under fluoroscopy as shown schematically in FIG. 9.

The other embodiments of support frames shown in Figs. 1c-g would form LA and DA similar to that shown in Figs. 8 and 9.

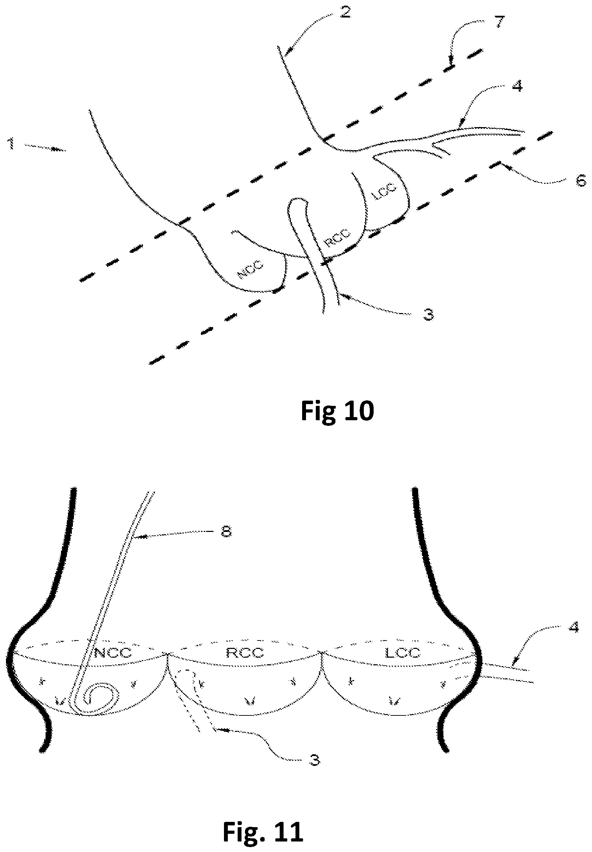

FIG. 10 depicts the aortic root complex 1. Aortic root is the dilated first part of aorta attached to the heart at its end. It is a part of the ascending aorta (AA) 2 containing the native aortic valve. There are generally three cusps of the native aortic valve. The coronary arteries originate from the vicinity of the aortic bulb from two of the three cusps. The Right Coronary Artery (RCA) 3 originates from Right Coronary Cusp (RCC) and the Left Coronary Artery (LCA) 4 originates from Left Coronary Cusp (LCC). The remaining cusp is termed Non-Coronary Cusp (NCC) as no coronary artery originates in the vicinity of this cusp. There are two distinct demarcation planes, one is the Virtual Annular Plane (VAP) 6 and the second is Sino tubular Junction (SJ) 7. In a standard procedure under fluoroscopic guidance, the native coronary cusps are made co-planar wherein the hinge point of each cusp is in a straight line and all the three cusps are well separated. The VAP 6 thus achieved can be visible under fluoroscopy and is a guiding feature to position the prosthetic valve at the optimal location for implantation.

During THV replacement procedure, under fluoroscopic guidance, the three native coronary cusps (sinuses) RCC, LCC, NCC are visually aligned in a co-planar view wherein non-coronary cusp (NCC) appears to the right of the patient and the left coronary cusp (LCC) appears to the left of the patient with the RCC lying in the center. This is shown schematically in Fig. 11. It may be noted that the fluoroscopic view is a mirror image of the true anatomical/AP view. Hence in the schematic image of Fig. 11, NCC appears to the right of the patient and the LCC appears to the left of the patient.

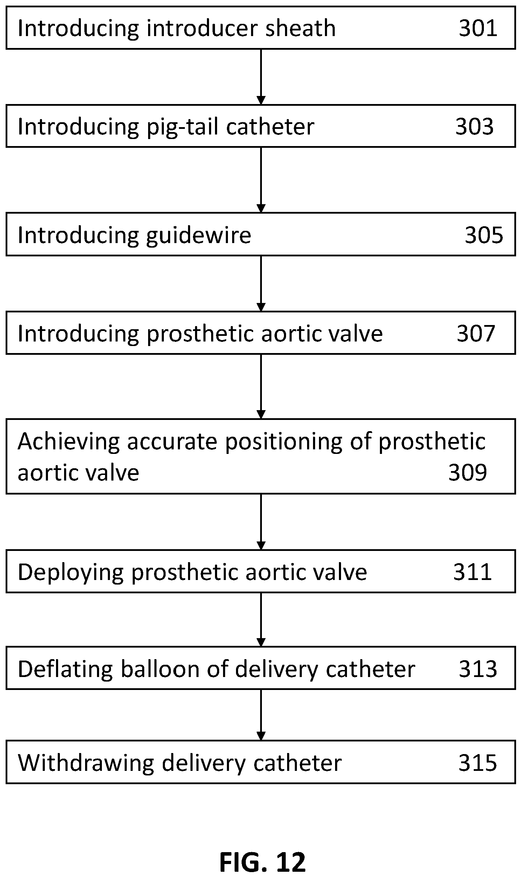

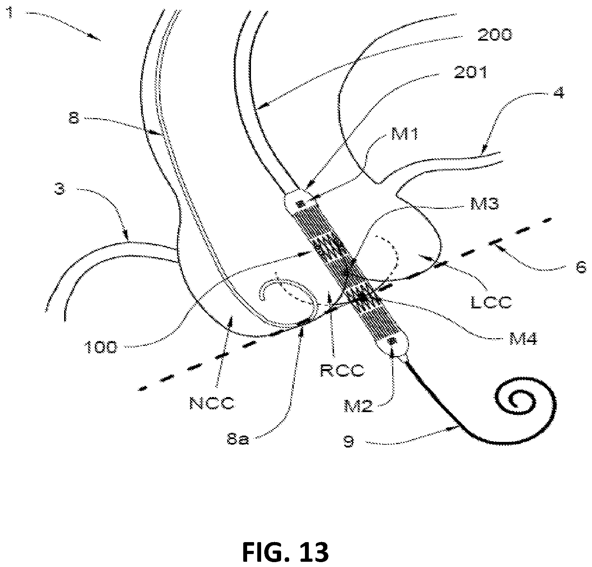

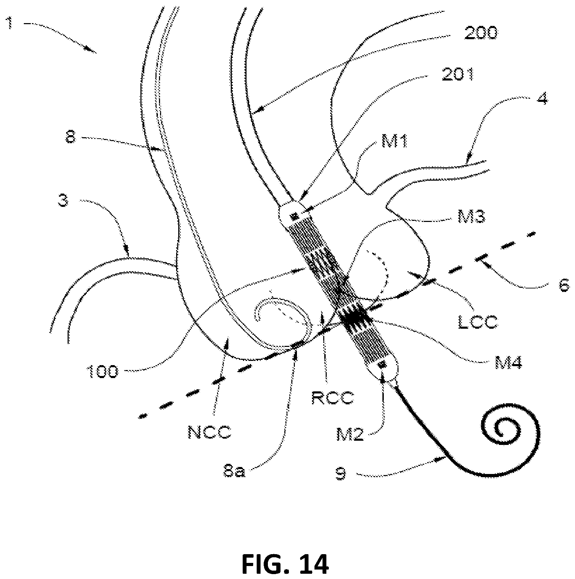

FIG. 12 describes a method (not claimed) of accurately positioning and deploying the prosthetic aortic valve 100 at an annular plane i.e. orthotopic position using the delivery catheter 200 in step-wise manner. Figs. 13 and 14 show schematically the implantation positions. Fig. 13 shows the THV 100 crimped on balloon 201 of the catheter 200 shown in Fig. 8 (corresponding to frame shown in Fig. 1b). Fig. 14 shows the THV 100 crimped on balloon 201 of the catheter 200 shown in Fig. 9 (corresponding to frame shown in Fig. 1c). These frame structures are shown only to demonstrate the implantation method. This method is applicable to any other frame structure described above.

A well-qualified operator trained in percutaneous implantation of heart valve generally introduces an introducer sheath into the vasculature of a patient at step 301. Subsequently at step 303, a standard angiographic pig-tail catheter 8 (preferably of 5F size) is introduced and navigated through the introducer sheath into the patient's vasculature and its distal end is parked at the lowest end within the NCC (considering that NCC is usually the lowest reference cusp and without any coronary artery originating) under fluoroscopic guidance. This arrangement is shown in Figs. 13 and 14 where the curved distal end 8a of the pig-tail catheter 8 rests within NCC. At step 305, a standard recommended guidewire 9 is introduced under fluoroscopic guidance and is navigated beyond the aortic orifice of the patient.

Upon injection of a saline diluted contrast medium through the pig-tail catheter 8, the operator is able to visualize the aortic root 1. The VAP is determined as the virtual line 6 that could be drawn joining the low points of the aortic root 1 (refer to Fig. 10).

At step 307, the THV 100 of the present invention pre-crimped on the delivery catheter 200, is introduced, guided and navigated beyond the aortic annulus of the patient over a standard recommended guidewire under fluoroscopic guidance.

At step 309, for accurate positioning of the THV 100 at the annular plane, the centre of the landing zone marker M4 within the balloon 201 of the THV delivery system 200 must coincide with the lower end 8a of the pigtail 8 placed within the lowest part of NCC (identifying the VAP 6) as shown in Figs. 13 and 14. In other words, the centre of the landing zone marker M4 coincides with the VAP 6. In the absence of the landing zone marker M4, accurate positioning of the THV 100 at the annular plane is achieved by coinciding the center of the LA towards the distal end of the frame 101 with the lower end 8a of the pigtail 8 placed within the lowest part of NCC (identifying the VAP 6). Thus, landing zone marker M4 is not absolutely necessary, but it acts only as a guide and eases the accurate placement. The THV 100 is coaxial to the virtual annular plane 6 and thus adjacent to the pigtail 8 as visualized under fluoroscopy. This ensures the THV 100 to attain desired pre-deployment position. This may be achieved by simultaneous injection of diluted contrast medium under fluoroscopy.

Alternatively, or in combination, the THV 100 may be positioned such that the center of the light area (LA) towards the inflow end 100a coincides either with the lower end 8a of the pigtail 8 placed within the lowest part of NCC or with the VAP 6 as shown in Figs. 13 and 14.

Once aforesaid annular position is attained, the THV 100 can be deployed by balloon inflation while rapid pacing the heart using standard known technique at step 311. Due to the previously described two row octagonal cell geometry of the present invention, the expansion of the frame 101 and its associated low foreshortening allow precise orthotopic deployment. The accuracy of deployment is enhanced due to lower foreshortening ratio of THV 100 due to less number of rows of cells and frame scaffold structure.

After deployment of the THV 100, rapid pacing is discontinued. At step 313, the balloon 201 is then quickly deflated and catheter 200 is withdrawn from the deployed THV 100 and subsequently out from the patient's body at step 315. Thereafter, the guidewire and pigtail catheter are also similarly withdrawn using standard techniques.

The foregoing description of preferred embodiments of the present disclosure provides illustration and description, but is not intended to be exhaustive or to limit the disclosure to the precise form disclosed. Modifications and variations are possible in light of the above teachings or may be acquired from practice of the disclosure.

For example, the frame may include more than two rows of tessellating octagonal cells placed one above the other formed by a plurality of circumferentially extending rows of angled struts. In such case, the frame may include an uppermost row of angled struts at the proximal end of the frame, a lowermost row of angled struts at the distal end of the frame and a plurality of intermediate rows of angled struts in between the two. The lowermost row is disposed towards an inflow end of the support frame. Further, in such embodiment, an upper row would be referred relative to the adjacent row below which will be referred to as the lower row.

In the above example, the circumferentially extending rows of angled struts may include an undulating shape with any two consecutive angled struts of a row of circumferentially extending angled struts form a peak or a valley. The peaks of an upper row of angled struts face the valleys of an adjacent lower row of angled struts.

The adjacent circumferentially extending rows of angled struts are connected to each other to form the support frame including a plurality of adjacently placed rows of cells between its distal end and the proximal end. The plurality of adjacently placed rows of cells includes an uppermost row of cells and a lower row of cells.

The valleys of an upper row of angled struts are connected to the corresponding peaks of an adjacent lower row of angled struts by links (either a diamond shaped cell or a rhombus body) thereby forming a row of cells that include interlaced octagonal cells creating alternate sequence of rhombus bodies or diamond shaped cells at each junction. The said rhombus body may include a solid structure and the diamond shaped cells have an open structure. The uppermost row of cells may include three rhombus bodies, spaced angularly with respect to each other forming three commissure attachment areas. The said rhombus bodies are provided with holes.

Owing to the possibility of having various arrangements of the said links in the different cell rows, a plurality of embodiments of the support frame may be obtained.

Such a prosthetic aortic valve may further include leaflets, an internal skirt and an external skirt like the prosthetic aortic valve as described above.

Such frame provides requisite columnar strength. The method of deploying such stent will accordingly be altered for proper positioning of the stent using a corresponding delivery system.

The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A prosthetic aortic valve (100) which is radially expandable and collapsible and suitable for mounting on a balloon of a delivery catheter in a radially collapsed condition, the prosthetic aortic valve (100) comprising:
• a radially collapsible and expandable support frame (101) having a distal end (100a), a proximal end (100b) and three circumferentially extending rows of angled struts (10a, 10b, 10c) comprising an upper row of angled struts (10a) at the proximal end (100a) of the support frame (101), a lower row of angled struts (10c) at the distal end (100b) of the support frame (101) and a middle row of angled struts (10b) located between the upper row of angled struts (10a), and the lower row of angled struts (10c), wherein, the lower row of angled struts (10c) is towards an inflow end (100b) of the support frame (101);
∘ wherein, the circumferentially extending rows of angled struts (10a, 10b, 10c) have an undulating shape with any two consecutive angled struts of a row of circumferentially extending angled struts form a peak or a valley, the peaks of the upper row of angled struts (10a) face the valleys of the middle row of angled struts (10b), and the peaks of the middle row of angled struts (10b) face the valleys of the lower row of angled struts (10c);
∘ the adjacent circumferentially extending rows of angled struts (10a, 10b, 10c) are connected to each other to form the support frame (101) including two adjacently placed rows of cells (101b) between its distal end (100a) and the proximal end (100b),
∘ wherein, the valleys of the upper row of angled struts (10a) are connected to the corresponding peaks of the middle row of angled struts (10b) by links, where each link forms either a diamond shaped cell (101c) or a rhombus body (101d/101c'), thereby forming an upper row of cells (10162) that comprises interlaced octagonal cells creating alternate sequence of rhombus bodies (101d/101c') or diamond shaped cells (101c) at each junction, wherein, the said rhombus body (101d/101c') has a solid structure and the diamond shaped cells (101c) have an open structure;
∘ wherein, the valleys of the middle row of angled struts (10b) are connected to the corresponding peaks of the lower row of angled struts (10c) by links where each link forms either a diamond shaped cell (101c) or a rhombus body (101d/101c'), thereby forming a lower row of cells (101b1), adjacent to the upper row of cells (10162) that comprises interlaced octagonal cells creating alternate sequence of diamond shaped cells (101c) or rhombus bodies (101d/101c') at each junction, wherein, the said diamond shaped cells (101c) have an open structure and the said rhombus bodies (101d/101c') have a solid structure;
∘ wherein, the lower row of cells (101b1) is towards the inflow end (100b) of the support frame (100) and the upper row of cells (101b2) is towards the outflow end (100a) of the support frame (101),
∘ wherein, the upper row of cells (10162) comprises of three rhombus bodies, spaced angularly with respect to each other forming three commissure attachment areas (101d) where commissure tabs of leaflets (103) are attached, wherein, the said rhombus bodies (101d) are provided with holes (101d1);
• three leaflets (103) made from a biocompatible material with sufficient flexibility to allow movement of the leaflets (103) that allows unidirectional flow of blood from an inflow end of the prosthetic aortic valve an outflow end and prevent the flow of blood in the reverse direction by opening and closing the leaflets (103);
• an internal skirt (105) made of a biocompatible material covering an internal surface of the lower row of cells (101b1) at least partially; and
• an external skirt (107) made of a biocompatible material covering an external surface of the lower row of cells (101b1) at least partially.

2. The prosthetic aortic valve (100) of claim 1 wherein, the cells of the support frame (101) in the upper row (10162) and the cells in the lower row (101b1) are of same size.

3. The prosthetic aortic valve (100) of claim 1 wherein, the cells of the support frame (100) in the upper row (10162) are one of, larger or smaller than the cells in the lower row (101b1) in size.

4. The prosthetic aortic valve (100) of claim 1 wherein, each link of the support frame (101) in the upper row of cells (10162), other than the three rhombus bodies with holes (101d), is a diamond shaped cell (101c) or a rhombus body without hole (101c') and wherein, each link in the lower row of cells (101b1) is at least one of, a diamond shaped cell (101c) or a rhombus body without hole (101c').

5. The prosthetic aortic valve (100) of claim 1 wherein:
each link of the support frame (100) in the upper row of cells (10162), other than the three rhombus bodies with holes (101d), is a rhombus body (101c'), and wherein, all the links in the lower row of cells (101b1) are diamond shaped cells (101c); or
each link of the support frame (100) in the upper row of cells (101b2), other than the three rhombus bodies with holes (101d), forms a diamond shaped cell (101c) and all the links in the lower row of cells (101b1) are rhombus bodies (101c'); or
all the links of the support frame (101) in the upper and lower rows of cells (10162, 101b1), other than the three rhombus bodies with holes (101d) in the upper row of cells (10162), form rhombus bodies (101c'); or
all the links of the support frame (101) in the upper and lower rows of cells (10162, 101b1), other than the three rhombus bodies with holes (101d) in the upper row of cells (10162), are diamond shaped cells (101c).

6. The prosthetic aortic valve (100) of claim 1 wherein, each of the leaflets (103) includes a relatively straight upper edge (103a) with or without an apex (103a1), one commissure tab (10361, 10362) at each side of the leaflet (103) at its upper edge (103a), and a scalloped lower edge (103c) attached to the internal skirt (105), wherein, the upper edge (103a) is kept free for coaptation.

7. The prosthetic aortic valve (100) of claim 1 wherein, the leaflets (103) comprise a relatively straight upper edge (103a) with or without an apex (103a1), one commissure tab (103b1, 10362) at each side of the leaflet (103) at upper edge (103a), and a straight lower edge (103c') and two side edges (103c") attached to the internal skirt (105), wherein, the upper edge (103a) is kept free for coaptation.

8. The prosthetic aortic valve (100) of claim 1 wherein, the biocompatible material used for making the leaflets (103) includes an animal tissue where the commissure tabs (10361, 103b2) on the sides of the leaflet (103) are attached to the support frame (101) either directly or through an intermediate fabric layer to prevent direct contact of the animal tissue with the metal of the frame (101).

9. The prosthetic aortic valve (100) of claim 8 wherein, the animal tissue includes bovine pericardium.

10. The prosthetic aortic valve (100) of claim 1 wherein, the biocompatible material used for making the leaflets (103) is a biocompatible polymeric synthetic material.

11. The prosthetic aortic valve (100) of claim 10 wherein, the synthetic polymeric material includes a fabric material.

12. The prosthetic aortic valve (100) of claim 1 wherein, at least one of the internal skirt (105) or the external skirt (107) is made of a fabric material or an animal tissue material.

13. The prosthetic aortic valve (100) of claim 1 wherein, the support frame (101) is made of a fluoroscopic material.

14. The prosthetic aortic valve (100) of claim 1 wherein, the support frame (100) is made from a metal or a metal alloy.

15. The prosthetic aortic valve (100) of claim 14 wherein, the metal alloy includes one of cobalt-chromium-nickel alloy or cobalt-chromium-nickel-molybdenum alloy MP35N.

16. The prosthetic aortic valve (100) of claim 1 wherein, the external skirt (107) includes excess material such that the external skirt (107) forms a slack when the support frame (100) is in a radially expanded condition and the slack reduces when the support frame (100) is in the radially collapsed condition.

17. The prosthetic aortic valve (100) of claim 1 wherein, the commissure attachment areas (101d) are spaced angularly at 120° with respect to each other.

18. An assembly comprising:
• a prosthetic aortic valve of any of the claims 1-17, and
• a delivery catheter having a proximal end (A) and a distal end (B), comprising:
∘ an elongated shaft (203) having a distal end and a proximal end;
∘ an inflatable balloon (201) attached to the distal end of the elongated shaft (203), and a handle (211) attached to the proximal end of the elongated shaft (203), wherein, the distal end refers to the end away from an operator, and
∘ a plurality of radiopaque markers provided on a portion of the elongated shaft of a delivery catheter that is located within the inflatable balloon; the plurality of radiopaque markers comprising at least a distal marker (M2), a proximal marker (M1), a middle marker (M3) and a landing zone marker (M4);
∘ wherein, the distal marker (M2) is located towards a distal end of the balloon (201), the proximal marker (M1) is located towards a proximal end of the balloon (201), the middle marker (M3) is located between the proximal and distal markers (M1, M2) equidistant from the distal and proximal markers (M2, M1), and the landing zone marker (M4) is located between the distal marker (M2) and the middle marker (M3)
wherein, the prosthetic aortic valve (100) has fluoroscopic properties and when crimped on the balloon (201) of the delivery catheter (200), exhibits alternate light and dense areas under fluoroscopy,
wherein, dense areas are formed by circumferentially extending rows of angled struts (10a, 10b, 10c) and light areas are formed by the links formed by the diamond shaped cells (101c) or the rhombus bodies (101c') and the commissure attachment areas (101d);
wherein, the landing zone marker (M4) of the delivery catheter (200) is located behind a mid-point of the light area towards the inflow end of the prosthetic aortic valve (100).

## Patentansprüche

1. Prothetische Aortenklappe (100), die radial expandierbar und einklappbar ist und dazu geeignet ist, in einem radial eingeklappten Zustand an einem Ballon eines Zuführkatheters montiert zu werden, wobei die prothetische Aortenklappe (100) Folgendes umfasst:
• einen radial einklappbaren und expandierbaren Stützrahmen (101), der ein distales Ende (100a), ein proximales Ende (100b) und drei sich in Umfangsrichtung erstreckende Reihen von abgewinkelten Streben (10a, 10b, 10c) aufweist, umfassend eine obere Reihe von abgewinkelten Streben (10a) am proximalen Ende (100a) des Stützrahmens (101), eine untere Reihe von abgewinkelten Streben (10c) am distalen Ende (100b) des Stützrahmens (101) und eine mittlere Reihe von abgewinkelten Streben (10b), die sich zwischen der oberen Reihe von abgewinkelten Streben (10a) und der unteren Reihe von abgewinkelten Streben (10c) befindet, wobei die untere Reihe von abgewinkelten Streben (10c) in Richtung eines Einlassendes (100b) des Stützrahmens (101) weist;
∘ wobei die sich in Umfangsrichtung erstreckenden Reihen von abgewinkelten Streben (10a, 10b, 10c) eine wellenförmige Form aufweisen, wobei jeweils zwei aufeinanderfolgende abgewinkelte Streben einer Reihe von sich in Umfangsrichtung erstreckenden abgewinkelten Streben einen Gipfel oder ein Tal bilden, wobei die Gipfel der oberen Reihe von abgewinkelten Streben (10a) den Tälern der mittleren Reihe von abgewinkelten Streben (10b) gegenüberliegen, und die Gipfel der mittleren Reihe von abgewinkelten Streben (10b) gegenüber den Tälern der unteren Reihe von abgewinkelten Streben (10c) liegen;
∘ wobei die angrenzenden, sich in Umfangsrichtung erstreckenden Reihen von abgewinkelten Streben (10a, 10b, 10c) miteinander verbunden sind, um den Stützrahmen (101) zu bilden, der zwei angrenzend angeordnete Reihen von Zellen (101b) zwischen seinem distalen Ende (100a) und dem proximalen Ende (100b) einschließt,
∘ wobei die Täler der oberen Reihe von abgewinkelten Streben (10a) mit den entsprechenden Gipfeln der mittleren Reihe von abgewinkelten Streben (10b) durch Verbindungen verbunden sind, wobei jede Verbindung entweder eine diamantförmige Zelle (101c) oder einen Rautenkörper (101d/101c') bildet, wodurch eine obere Reihe von Zellen (10162) gebildet wird, die ineinander verschachtelte achteckige Zellen umfasst, die an jeder Verbindung eine abwechselnde Sequenz von Rautenkörpern (101d/101c') oder diamantförmigen Zellen (101c) erzeugen, wobei der Rautenkörper (101d/101c') eine feste Struktur aufweist und die diamantförmigen Zellen (101c) eine offene Struktur aufweisen;
∘ wobei die Täler der mittleren Reihe von abgewinkelten Streben (10b) mit den entsprechenden Gipfeln der unteren Reihe von abgewinkelten Streben (10c) durch Verbindungen verbunden sind, wobei jede Verbindung entweder eine diamantförmige Zelle (101c) oder einen Rautenkörper (101d/101c') bildet, wodurch eine untere Reihe von Zellen (101b1) gebildet wird, angrenzend an die obere Reihe von Zellen (10162), die verschachtelte achteckige Zellen umfasst, die an jeder Verbindung eine abwechselnde Sequenz von diamantförmigen Zellen (101c) oder Rautenkörpern (101d/101c') bilden, wobei die diamantförmigen Zellen (101c) eine offene Struktur aufweisen und die Rautenkörper (101d/101c') eine feste Struktur aufweisen;
∘ wobei die untere Reihe von Zellen (101b1) zum Einlassende (100b) des Stützrahmens (100) hin und die obere Reihe von Zellen (101b2) zum Auslassende (100a) des Stützrahmens (101) hin gerichtet ist,
∘ wobei die obere Reihe von Zellen (10162) drei Rautenkörper umfasst, die in einem Winkel zueinander beabstandet sind und drei Kommissurbefestigungsbereiche (101d) bilden, an denen Kommissurlaschen von Segeln (103) befestigt sind, wobei die Rautenkörper (101d) mit Löchern (101d1) versehen sind;
• drei Segel (103), gebildet aus einem biokompatiblen Material mit ausreichender Flexibilität, um eine Bewegung der Segel (103) zu ermöglichen, die einen unidirektionalen Blutfluss von einem Einlassende der prothetischhen Aortenklappe zu einem Auslassende ermöglicht und den Blutfluss in umgekehrter Richtung durch Öffnen und Schließen der Segel (103) verhindert;
• eine innere Schürze (105), bestehend aus einem biokompatiblen Material, das eine innere Oberfläche der unteren Reihe von Zellen (101b1) mindestens teilweise bedeckt; und
• eine äußere Schürze (107), bestehend aus einem biokompatiblen Material, das eine äußere Oberfläche der unteren Reihe von Zellen (101b1) mindestens teilweise bedeckt.

2. Prothetische Aortenklappe (100) nach Anspruch 1, wobei die Zellen des Stützrahmens (101) in der oberen Reihe (10162) und die Zellen in der unteren Reihe (101b1) die gleiche Größe aufweisen.

3. Prothetische Aortenklappe (100) nach Anspruch 1, wobei die Zellen des Stützrahmens (100) in der oberen Reihe (10162) größer oder kleiner als die Zellen in der unteren Reihe (101b1) sind.

4. Prothetische Aortenklappe (100) nach Anspruch 1, wobei jedes Glied des Stützrahmens (101) in der oberen Reihe von Zellen (10162), mit Ausnahme der drei Rautenkörper mit Löchern (101d), eine diamantförmige Zelle (101c) oder ein Rautenkörper ohne Loch (101c') ist und wobei jedes Glied in der unteren Reihe von Zellen (101b1) mindestens eines von einer diamantförmigen Zelle (101c) oder einem Rautenkörper ohne Loch (101c') ist.

5. Prothetische Aortenklappe (100) nach Anspruch 1, wobei:
jedes Glied des Stützrahmens (100) in der oberen Reihe von Zellen (10162), mit Ausnahme der drei Rautenkörper mit Löchern (101d), ein Rautenkörper (101c') ist, und wobei alle Glieder in der unteren Reihe von Zellen (101b1) diamantförmige Zellen (101c) sind; oder
jedes Glied des Stützrahmens (100) in der oberen Reihe von Zellen (101b2), mit Ausnahme der drei Rautenkörper mit Löchern (101d), eine diamantförmige Zelle (101c) bildet und alle Glieder in der unteren Reihe von Zellen (101b1) Rautenkörper (101c') sind; oder
alle Glieder des Stützrahmens (101) in der oberen und der unteren Reihe von Zellen (10162, 101b1), mit Ausnahme der drei mit Löchern versehenen Rautenkörper (101d) in der oberen Reihe von Zellen (10162), Rautenkörper (101c') bilden; oder
alle Glieder des Stützrahmens (101) in der oberen und der unteren Reihe von Zellen (10162, 101b1), mit Ausnahme der drei mit Löchern versehenen Rautenkörper (101d) in der oberen Reihe von Zellen (10162), diamantförmige Zellen (101c) sind.

6. Prothetische Aortenklappe (100) nach Anspruch 1, wobei jedes der Segel (103) einen relativ geraden oberen Rand (103a) mit oder ohne Apex (103a1), eine Kommissurlasche (10361, 10362) an jeder Seite des Segels (103) an seinem oberen Rand (103a) und einen gewellten unteren Rand (103c) einschließt, der an der inneren Schürze (105) befestigt ist, wobei der obere Rand (103a) für die Koaptation frei gehalten wird.

7. Prothetische Aortenklappe (100) nach Anspruch 1, wobei die Segel (103) einen relativ geraden oberen Rand (103a) mit oder ohne Apex (103a1), eine Kommisurlasche (103b1, 10362) an jeder Seite des Segels (103) am oberen Rand (103a) und einen geraden unteren Rand (103c') und zwei seitliche Ränder (103c") umfassen, die an der inneren Schürze (105) befestigt sind, wobei der obere Rand (103a) für die Koaptation frei gehalten wird.

8. Prothetische Aortenklappe (100) nach Anspruch 1, wobei das biokompatible Material, das für die Herstellung der Segel (103) verwendet wird, ein tierisches Gewebe einschließt, worin die Kommisurlaschen (10361, 103b2) an den Seiten der Segel (103) entweder direkt oder durch eine dazwischenliegende Stoffschicht an dem Stützrahmen (101) befestigt sind, um einen direkten Kontakt des tierischen Gewebes mit dem Metall des Rahmens (101) zu verhindern.

9. Prothetische Aortenklappe (100) nach Anspruch 8, wobei das tierische Gewebe Rinderperikard einschließt.

10. Prothetische Aortenklappe (100) nach Anspruch 1, wobei das biokompatible Material, das zur Herstellung der Segel (103) verwendet wird, ein biokompatibles polymeres synthetisches Material ist.

11. Prothetische Aortenklappe (100) nach Anspruch 10, wobei das synthetische polymere Material ein Gewebematerial einschließt.

12. Prothetische Aortenklappe (100) nach Anspruch 1, wobei mindestens eine aus entweder der inneren Schürze (105) oder der äußeren Schürze (107) aus einem Gewebematerial oder einem tierischen Gewebematerial besteht.

13. Prothetische Aortenklappe (100) nach Anspruch 1, wobei der Stützrahmen (101) aus einem fluoroskopischen Material besteht.

14. Prothetische Aortenklappe (100) nach Anspruch 1, wobei der Stützrahmen (100) aus einem Metall oder einer Metalllegierung gebildet ist.

15. Prothetische Aortenklappe (100) nach Anspruch 14, wobei die Metalllegierung eine aus einer Kobalt-Chrom-Nickel-Legierung oder einer Kobalt-Chrom-Nickel-MolybdänLegierung MP35N einschließt.

16. Prothetische Aortenklappe (100) nach Anspruch 1, wobei die äußere Schürze (107) überschüssiges Material einschließt, sodass die äußere Schürze (107) eine Schlaufe bildet, wenn sich der Stützrahmen (100) in einem radial expandierten Zustand befindet, und die Schlaufe sich verringert, wenn sich der Stützrahmen (100) in einem radial eingeklappten Zustand befindet.

17. Prothetische Aortenklappe (100) nach Anspruch 1, wobei die Kommissurbefestigungsbereiche (101d) in einem Winkel von 120° in Bezug aufeinander beabstandet sind.

18. Anordnung, Folgendes umfassend:
• eine prothetische Aortenklappe nach einem der Ansprüche 1 bis 17, und
• einen Zuführkatheter mit einem proximalen Ende (A) und einem distalen Ende (B), Folgendes umfassend:
∘ einen länglichen Schaft (203), der ein distales Ende und ein proximales Ende aufweist;
∘ einen aufblasbaren Ballon (201), der am distalen Ende des länglichen Schafts (203) befestigt ist, und einen Griff (211), der am proximalen Ende des länglichen Schafts (203) befestigt ist, wobei sich das distale Ende auf das Ende bezieht, das von einem Bediener entfernt ist, und
∘ eine Vielzahl von röntgendichten Markierungen, die auf einem Abschnitt des länglichen Schafts eines Zuführkatheters bereitgestellt sind, der sich innerhalb des aufblasbaren Ballons befindet; wobei die Vielzahl von röntgendichten Markierungen mindestens eine distale Markierung (M2), eine proximale Markierung (M1), eine mittlere Markierung (M3) und eine Landezonenmarkierung (M4) umfasst;
∘ wobei die distale Markierung (M2) in Richtung eines distalen Endes des Ballons (201) positioniert ist, die proximale Markierung (M1) in Richtung eines proximalen Endes des Ballons (201) positioniert ist, die mittlere Markierung (M3) zwischen der proximalen und der distalen Markierung (M1, M2) in gleichem Abstand von der distalen und der proximalen Markierung (M2, M1) positioniert ist und die Landezonenmarkierung (M4) zwischen der distalen Markierung (M2) und der mittleren Markierung (M3) positioniert ist,
wobei die prothetische Aortenklappe (100) fluoroskopische Eigenschaften aufweist und, wenn sie auf den Ballon (201) des Zuführkatheters (200) gecrimpt ist, unter Durchleuchtung abwechselnd helle und dichte Bereiche aufweist,
wobei die dichte Bereiche durch sich in Umfangsrichtung erstreckende Reihen von abgewinkelten Streben (10a, 10b, 10c) gebildet werden und die helle Bereiche durch die Verbindungen gebildet werden, die durch die diamantförmigen Zellen (101c) oder die Rautenkörper (101c') und die Kommissurbefestigungsbereiche (101d) gebildet werden;
wobei die Landezonenmarkierung (M4) des Einführkatheters (200) hinter einem Mittelpunkt des hellen Bereichs in Richtung des Einlassendes der prothetischen Aortenklappe (100) positioniert ist.

## Revendications

1. Valve aortique prothétique (100) pouvant être étendue et repliée radialement et convenant pour être montée sur un ballonnet d'un cathéter d'introduction dans un état replié radialement, la valve aortique prothétique (100) comprenant :
• une structure de support pouvant être repliée et étendue radialement (101) comportant une extrémité distale (100a), une extrémité proximale (100b) et trois rangées de contrefiches inclinées (10a, 10b, 10c) s'étendant de façon circonférentielle, comprenant une rangée supérieure de contrefiches inclinées (10a) au niveau de l'extrémité proximale (100a) de la structure de support (101), une rangée inférieure de contrefiches inclinées (10c) au niveau de l'extrémité distale (100b) de la structure de support (101) et une rangée intermédiaire de contrefiches inclinées (10b) située entre la rangée supérieure de contrefiches inclinées (10a) et la rangée inférieure de contrefiches inclinées (10c), dans laquelle la rangée inférieure de contrefiches inclinées (10c) est orientée en direction d'une extrémité d'entrée (100b) de la structure de support (101) ;
∘ dans laquelle les rangées de contrefiches inclinées (10a, 10b, 10c) s'étendant de façon circonférentielle présentent une forme ondulée, deux quelconques contrefiches inclinées consécutives d'une rangée de contrefiches inclinées s'étendant de façon circonférentielle forment une crête ou une vallée, les crêtes de la rangée supérieure de contrefiches inclinées (10a) font face aux vallées de la rangée intermédiaire de contrefiches inclinées (10b), et les crêtes de la rangée intermédiaire de contrefiches inclinées (10b) font face aux vallées de la rangée inférieure de contrefiches inclinées (10c) ;
∘ les rangées adjacentes de contrefiches inclinées (10a, 10b, 10c) s'étendant de façon circonférentielle sont mutuellement connectées pour former la structure de support (101) incluant deux rangées positionnées de façon adjacente de cellules (101b) entre son extrémité distale (100a) et son extrémité proximale (100b),
∘ dans laquelle les vallées de la rangée supérieure de contrefiches inclinées (10a) sont connectées aux crêtes correspondantes de la rangée intermédiaire de contrefiches inclinées (10b) par des éléments de liaison, chaque élément de liaison formant soit une cellule en forme de losange (101c), soit un corps rhomboïde (101d/101c'), formant ainsi une rangée supérieure de cellules (10162) qui comprend des cellules octogonales entrelacées créant une séquence alternée de corps rhomboïdes (101d/101c') ou de cellules en forme de losange (101c) au niveau de chaque jonction, dans laquelle lesdits corps rhomboïdes (101d/101c') présentent une structure solide et les cellules en forme de losange (101c) présentent une structure ouverte ;
∘ dans laquelle les vallées de la rangée intermédiaire de contrefiches inclinées (10b) sont connectées aux crêtes correspondantes de la rangée inférieure de contrefiches inclinées (10c) par des éléments de liaison, chaque élément de liaison formant soit une cellule en forme de losange (101c), soit un corps rhomboïde (101d/101c'), formant ainsi une rangée inférieure de cellules (101b1) adjacente à la rangée supérieure de cellules (10162) qui comprend des cellules octogonales entrelacées créant une séquence alternée de cellules en forme de losange (101c) ou de corps rhomboïdes (101d/101c') au niveau de chaque jonction, dans laquelle lesdites cellules en forme de losange (101c) présentent une structure ouverte et lesdits corps rhomboïdes (101d/101c') présentent une structure solide ;
∘ dans laquelle la rangée inférieure de cellules (101b1) est orientée en direction de l'extrémité d'entrée (100b) de la structure de support (100) et la rangée supérieure de cellules (101b2) est orientée en direction de l'extrémité de sortie (100a) de la structure de support (101),
∘ dans laquelle la rangée supérieure de cellules (10162) comprend trois corps rhomboïdes mutuellement espacés angulairement et qui forment trois zones de liaison de type commissure (101d) au niveau desquelles des languettes de type commissure de folioles (103) sont liées, dans laquelle lesdits corps rhomboïdes (101d) sont munis de trous (101d1) ;
• trois folioles (103) réalisées en un matériau biocompatible et présentant une flexibilité suffisante pour permettre un déplacement des folioles (103) qui permet un écoulement unidirectionnel de sang depuis une extrémité d'entrée de la valve aortique prothétique jusqu'à une extrémité de sortie et qui empêche l'écoulement de sang en sens inverse en ouvrant et en fermant les folioles (103) ;
• une jupe interne (105) constituée d'un matériau biocompatible et recouvrant une surface interne de la rangée inférieure de cellules (101b1) au moins partiellement ; et
• une jupe externe (107) constituée d'un matériau biocompatible et recouvrant une surface externe de la rangée inférieure de cellules (101b1) au moins partiellement.

2. Valve aortique prothétique (100) selon la revendication 1, dans laquelle les cellules de la structure de support (101) dans la rangée supérieure (10162) et les cellules dans la rangée inférieure (101b1) sont de la même dimension.

3. Valve aortique prothétique (100) selon la revendication 1, dans laquelle les cellules de la structure de support (100) dans la rangée supérieure (10162) sont soit plus grande, soit plus petite que les cellules dans la rangée inférieure (101b1).

4. Valve aortique prothétique (100) selon la revendication 1, dans laquelle chaque élément de liaison de la structure de support (101) dans la rangée supérieure de cellules (10162), autre que les trois corps rhomboïdes munis de trous (101d), est une cellule en forme de losange (101c) ou un corps rhomboïde sans trou (101c') et dans laquelle chaque élément de liaison dans la rangée inférieure de cellules (101b1) est au moins une parmi une cellule en forme de losange (101c) ou un corps rhomboïde sans trou (101c').

5. Valve aortique prothétique (100) selon la revendication 1, dans laquelle :
chaque élément de liaison de la structure de support (100) dans la rangée supérieure de cellules (10162), autre que les trois corps rhomboïdes munis de trous (101d), est un corps rhomboïde (101c'), et dans laquelle toutes les éléments de liaison dans la rangée inférieure de cellules (101b1) sont des cellules en forme de losange (101c) ; ou
chaque élément de liaison de la structure de support (100) dans la rangée supérieure de cellules (101b2), autre que les trois corps rhomboïdes (101d), forme une cellule en forme de losange (101c) et tous les éléments de liaison dans la rangée inférieure de cellules (101b1) sont des corps rhomboïdes (101c') ; ou
tous les éléments de liaison de la structure de support (101) dans les rangées supérieure et inférieure de cellules (10162, 101b1), autres que les trois corps rhomboïdes munis de trous (101d) dans la rangée supérieure de cellules (10162), forment des corps rhomboïdes (101c') ; ou
tous les éléments de liaison de la structure de support (101) dans les rangées supérieure et inférieure de cellules (10162, 101b1), autres que les trois corps rhomboïdes munis de trous (101d) dans la rangée supérieure de cellules (10162), sont des cellules en forme de losange (101c).

6. Valve aortique prothétique (100) selon la revendication 1, dans laquelle chacune des folioles (103) inclut un bord supérieur relativement rectiligne (103a) avec ou sans sommet (103a1), une languette de type commissure (10361, 10362) au niveau de chaque côté de la foliole (103) au niveau de son bord supérieur (103a) et un bord inférieur dentelé (103c) lié à la jupe interne (105), dans laquelle le bord supérieur (103a) est maintenu libre pour la coaptation.

7. Valve aortique prothétique (100) selon la revendication 1, dans laquelle les folioles (103) comprennent un bord supérieur relativement rectiligne (103a) avec ou sans sommet (103a1), une languette de type commissure (103b1, 10362) au niveau de chaque côté de la foliole (103) au niveau de son bord supérieur (103a) et un bord inférieur rectiligne (103c') et deux bords latéraux (103c") liés à la jupe interne (105), dans laquelle le bord supérieur (103a) est maintenu libre pour la coaptation.

8. Valve aortique prothétique (100) selon la revendication 1, dans laquelle le matériau biocompatible utilisé pour réaliser les folioles (103) inclut un tissu animal, où les languettes de type commissure (10361, 103b2) sur les côtés de la foliole (103) sont liées à la structure de support (101) soit directement, soit par l'intermédiaire d'une couche de tissu intermédiaire pour empêcher un contact direct du tissu animal avec le métal de la structure de support (101).

9. Valve aortique prothétique (100) selon la revendication 8, dans laquelle le tissu animal inclut du péricarde bovin.

10. Valve aortique prothétique (100) selon la revendication 1, dans laquelle le matériau biocompatible utilisé pour réaliser les folioles (103) est un matériau de synthèse polymérique biocompatible.

11. Valve aortique prothétique (100) selon la revendication 10, dans laquelle le matériau polymérique de synthèse inclut un matériau de tissu.

12. Valve aortique prothétique (100) selon la revendication 1, dans laquelle au moins une jupe de la jupe interne (105) ou de la jupe externe (107) est réalisée en un matériau de tissu ou en un matériau de tissu animal.

13. Valve aortique prothétique (100) selon la revendication 1, dans laquelle la structure de support (101) est constituée d'un matériau fluoroscopique.

14. Valve aortique prothétique (100) selon la revendication 1, dans laquelle la structure de support (100) est réalisée à partir d'un métal ou d'un alliage métallique.

15. Valve aortique prothétique (100) selon la revendication 14, dans laquelle l'alliage métallique inclut un alliage parmi un alliage cobalt-chrome-nickel ou un alliage cobalt-chrome-nickel-molybdène MP35N.

16. Valve aortique prothétique (100) selon la revendication 1, dans laquelle la jupe externe (107) inclut du matériau en excès de telle sorte que la jupe externe (107) forme un relâchement lorsque la structure de support (100) est dans un état étendu radialement et que le relâchement soit réduit lorsque la structure de support (100) est dans l'état replié radialement.

17. Valve aortique prothétique (100) selon la revendication 1, dans laquelle les zones de liaison de type commissure (101d) sont mutuellement espacées angulairement de 120°.

18. Assemblage comprenant :
• une valve aortique prothétique selon l'une quelconque des revendications 1 à 17 ; et
• un cathéter d'introduction comportant une extrémité proximale (A) et une extrémité distale (B), comprenant :
∘ un arbre allongé (203) comportant une extrémité distale et une extrémité proximale ;
∘ un ballonnet gonflable (201) lié à l'extrémité distale de l'arbre allongé (203), et une poignée (211) liée à l'extrémité proximale de l'arbre allongé (203), dans lequel l'extrémité distale désigne l'extrémité à distance d'un opérateur, et
∘ une pluralité de marqueurs radio-opaques prévus sur une partie de l'arbre allongé d'un cathéter d'introduction qui est situé à l'intérieur du ballonnet gonflable ; la pluralité de marqueurs radio-opaques comprenant au moins un marqueur distal (M2), un marqueur proximal (M1), un marqueur intermédiaire (M3) et un marqueur de zone d'aide au positionnement (M4) ;
∘ dans lequel le marqueur distal (M2) est situé en direction d'une extrémité distale du ballonnet (201), le marqueur proximal (M1) est situé en direction d'une extrémité proximale du ballonnet (201), le marqueur intermédiaire (M3) est situé entre les marqueurs proximal et distal (M1, M2) à équidistance des marqueurs distal et proximal (M2, M1), et le marqueur de zone d'aide au positionnement (M4) est situé entre le marqueur distal (M2) et le marqueur intermédiaire (M3) ;
dans lequel la valve aortique prothétique (100) présente des propriétés fluoroscopiques et lorsqu'elle est sertie sur le ballonnet (201) du cathéter d'introduction (200), elle présente des zones alternées claires et opaques sous fluoroscopie ;
dans lequel les zones opaques sont formées par des rangées de contrefiches inclinées (10a, 10b, 10c) s'étendant de façon circonférentielle et les zones légères sont formées par les éléments de liaison formés par les cellules en forme de losange (101c) ou les corps rhomboïdes (101c') et les zones de liaison de type commissure (101d) ;
dans lequel le marqueur de zone de positionnement (M4) du cathéter d'introduction (200) est situé derrière un point intermédiaire de la zone claire en direction de **l'extrémité** d'entrée de la valve aortique prothétique (100).
